# FASCICULE DE BREVET EUROPEEN

(11) **EP 1 527 089 B1**
(45) Date de publication et mention de la délivrance du brevet: **26.03.2008**
(21) Numéro de dépôt: 03769526.9
(22) Date de dépôt: 01.08.2003
(51) Int. Cl.: C07K 14/18

(54) **MELANGE DE PEPTIDES ISSUS DES PROTEINES C ET NS3 DU VIRUS DE HEPATITE C ET LEURS APPLICATIONS.**
MISCHUNG VON PEPTIDEN AUS DEN PROTEINEN C UND NS3 DES HEPATITIS C-VIRUS, UND DEREN VERWENDUNG
MIXTURE OF PEPTIDES FROM C AND NS3 PROTEINS OF THE HEPATITIS C VIRUS AND APPLICATIONS THEREOF

(30) Priorité: 02.08.2002 FR 0209874
(43) Date de publication de la demande: 04.05.2005
(73) Titulaire: COMMISSARIAT A L'ENERGIE ATOMIQUE, 75015 Paris (FR)
(72) Inventeur: MAILLERE, Bernard, F-78000 Versailles (FR); GEORGES, Bertrand, F-59221 Bauvin (FR); CASTELLI, Florence, F-92100 Montrouge (FR); BOUZIDI, M. Ahmed, F-59112 Annoeullin (FR)
(74) Mandataire: Vialle-Presles, Marie José
(86) Numéro de dépôt international: PCT/FR2003/002446
(87) Numéro de publication internationale: WO 2004/014936

(56) Documents cités:
- WO-A-02/34770
- WO-A-95/27733
- LAMONACA V ET AL: "CONSERVED HEPATITIS C VIRUS SEQUENCES ARE HIGHLY IMMUNOGENIC FOR CD4+ T CELLS: IMPLICATIONS FOR VACCINE DEVELOPMENT" , HEPATOLOGY, WILLIAMS AND WILKINS, BALTIMORE, MD, US, VOL. 30, NR. 4, PAGE(S) 1088-1098 XP001027157 ISSN: 0270-9139 abrégé page 1088, alinéa 2 page 1089, colonne 2, alinéa 4 -page 1090, colonne 1, alinéa 1 page 1094, colonne 1, alinéa 9 -page 1095, colonne 1, alinéa 2 page 1097, colonne 1, alinéa 1 page 1097, colonne 2, alinéa 3 page 1098, alinéa 1 tableaux 2,3 figure 4
- DIEPOLDER H M ET AL: "Possible mechanism involving T-lymphocyte response to non-structural protein 3 in viral clearance in acute hepatitis C virus infection" , THE LANCET, VOL. 346, PAGE(S) 1006-1007, 14 OCTOBRE 1995 XP001147781 ISSN: 0270-9139 le document en entier
- HITOMI Y ET AL: "HIGH EFFICIENCY PROKARYOTIC EXPRESSION AND PURIFICATION OF A PROTION OF THE HEPATITIS C CORE PROTEIN AND ANALYSIS OF THE IMMUNE RESPONSE TO RECOMBINANT PROTEIN IN BALB/C MICE" , VRAL IMMUNOLOGY, VOL. 8, NR. 2, PAGE8S9 109-119, 1995 XP000978724 ISSN: 0270-9139 le document en entier

## Description

La présente invention est relative à un mélange de peptides issus des protéines C et NS3 du virus de l'hépatite C (VHC) ainsi qu'à ses applications en tant que médicament (dans des compositions immunogènes, aptes à stimuler la production de lymphocytes T CD4+ anti-VHC *in vivo* et donc utiles pour la prévention et le traitement des infections à VHC) ou en tant que réactif de diagnostic de lymphocytes T spécifiques du VHC, notamment pour évaluer l'état immunitaire des patients.

Le virus de l'hépatite C (VHC) a été identifié en 1989 comme étant l'agent étiologique majeur des hépatites non-A non-B (Kato et al., P.N.A.S., 1990, 87, 9524-). L'homme est le seul hôte connu du VHC mais le virus peut être transmis expérimentalement aux chimpanzés. Le VHC infecte les hépatocytes, mais également certaines cellules du système immunitaire comme les lymphocytes, les monocytes et les cellules dendritiques.

La transmission du virus est essentiellement parentérale. Toutefois, comme les infections de *novo* sont rarement décelables, la voie de contamination demeure inconnue dans plus de 40% des cas. La prévalence de l'hépatite C est élevée dans la population générale, de l'ordre de 1 à 2% en Afrique, en Amérique, en Europe ainsi qu'en Asie du sud-est (Alter et al., Blood, 1995, 85, 1681-). Elle pourrait atteindre 5% dans certaines régions de Chine et dans le pacifique-ouest. Au Moyen-Orient, la prévalence varie de 1 jusqu'à 12%. Ainsi, il est estimé que 170 millions d'individus dans le monde sont porteurs du VHC.

Le VHC fait partie du genre *Hepacivirus* au sein de la famille des *Flaviviridae.* C'est un virus à ARN brin positif de 9,4 kb. Le génome possède un cadre de lecture ouvert principal qui code pour un long polypeptide clivable en 8 protéines. La protéine de capside (Core ou C, 191 acides aminés) possède une capacité de liaison à l'ARN viral et constitue la nucléocapside virale. Elle participe également à des effets cytopathogènes directs. Les glycoprotéines E1 et E2 sont insérées dans l'enveloppe du virus et impliquées dans les interactions avec les cellules hôtes notamment via le récepteur CD81. La protéine NS2 possède une fonction métalloprotéase impliquée notamment dans le clivage NS2/NS3. NS3 (631 acides aminés, le premier acide aminé de la protéine NS3 correspond à celui situé en position 1007 dans la séquence de la polyprotéine du VHC) et NS4 participent à la fois à une activité sérine protéase et ARN hélicase. Les protéines NS5A et NS5B sont des polymérases à ARN participant à la réplication du génome du VHC. Dans ce qui suit, la numérotation des acides aminés est indiquée en référence à la séquence de la polyprotéine du VHC de génotype la (SWISSPROT P26664 et Choo et al., P.N.A.S., 1991, 88, 2451-2455) ; il y a lieu de noter que cette numérotation est identique pour tous les génotypes du VHC dans la mesure où leurs polyprotéines présentent la même taille.

Le VHC peut être classifié en 6 génotypes ou clades (désignés de 1-6) et jusqu'à 100 sous-types (désignés par a, b, c, etc). Cette classification selon Simmonds et al. (J. Gen. Virol., 1993, 74, 2391-) est basée sur une analyse phylogénétique des séquences NS5. La variabilité inter-génotypique affecte également, mais à différents degrés, les autres protéines virales. Les génotypes 1-3 ont une distribution mondiale, les génotypes 4 et 5 sont présents principalement en Afrique tandis que le génotype 6 est principalement distribué en Asie. Au sein d'un individu infecté, les VHC ne représentent pas une espèce homogène mais constituent de nombreuses quasi-espèces. Ce phénomène est le résultat de l'apparition de mutations au sein du génome dues à la faible fidélité de réplication de la polymérase virale.

Le VHC occasionne chez l'homme des infections aiguës pouvant se résoudre naturellement mais peut conduire dans plus de 80% des cas à la persistance du virus. La phase aiguë est relativement bénigne avec seulement 20 à 30% des personnes infectées développant des symptômes ou des signes cliniques. Environ 60% des patients ont une augmentation des transaminases témoignant d'une hépatite chronique. Les études épidémiologiques indiquent que 20% des individus porteurs du virus développent une cirrhose après une vingtaine d'années suivie d'une décompensation hépatique, voire d'un hépatocarcinome. Dans les pays industrialisés, le VHC est responsable au total de 20% des hépatites aiguës, de 60% des carcinomes hépato-cellulaires et de 30% des transplantations hépatiques. Les traitements actuels (interféron-alpha/ribavirine) permettent la guérison d'environ 50% des patients en phase chronique.

Contrairement à l'infection par le VIH, l'infection par le VHC n'est pas systématiquement persistante. De nombreux patients infectés arrivent en effet à surmonter l'infection grâce à une réponse immunitaire efficace. Des lymphocytes T auxiliaires (T helper) pourraient être impliqués dans le contrôle précoce de la réplication virale (Thimme et al; J Exp. Med., 2001, 194, 1395). En effet, une forte activité des lymphocytes T auxiliaires, spécifiques des protéines C, NS3, NS4 et NS5, et de type Th1 (c'est-à-dire sécrétant des interleukines telles que l'IL2 et l'IFNγ) est associée au processus de guérison spontanée (Diepolder et al., Lancet, 1995, 346, 1006-; Pape et al., J. Virol. Hepat. Suppl., 1999, 1, 36; Gerlach et al., Gastroenterology, 1999, 117, 933). A l'inverse, une réponse T helper Th2 (c'est-à-dire caractérisée par la sécrétion d'IL4 et d'IL5) est associée à un mauvais pronostic de l'évolution de l'infection à VHC (Tai et al., J. Biomed. Sci., 2001, 8, 321). L'implication des lymphocytes T auxiliaires dans l'évolution de l'infection à VHC a été confortée par des analyses immunogénétiques révélant une corrélation entre la guérison spontanée et les allèles HLA de classe II (HLA-DRB1*1101/DQB1*03 ; Thurzs et al., J. Virol. Hepat., 1997, 4,215).

En revanche, chez les patients infectés chroniques, un nombre restreint d'épitopes T sont capables de restimuler, *in vitro,* les lymphocytes T issus du sang périphérique ou de prélèvements hépatiques et il est donc difficile de mesurer la production d'interféron γ par les lymphocytes T auxiliaires, après stimulation par les protéines virales (Core, NS3, NS4 et NS5). Il semblerait que l'orientation de la réponse immunitaire vers un profil du type Th0/Th2, ainsi qu'une tolérance aux antigènes viraux soient à l'origine de cette perte de l'immunité anti-virale et donc de la persistance du VHC.

Les réponses cellulaires médiées par les lymphocytes T CD4⁺ auxiliaires sont très souvent impliquées dans les mécanismes anti-viraux. Spécifiques des antigènes, les lymphocytes T CD4⁺ sont en effet capables de détecter la présence d'un agent pathogène et sous l'effet de cette reconnaissance, de déclencher une réponse immunitaire. La reconnaissance des antigènes conduit en effet à leur activation. Une fois activés, les lymphocytes T CD4+ sécrètent la plupart des cytokines nécessaires au recrutement de cellules effectrices que sont les lymphocytes CD8+ cytotoxiques et les lymphocytes B producteurs d'anticorps. Ils interviennent également dans l'activation des cellules par des contacts cellulaires et par exemple induisent l'activation par le CD40 des cellules dendritiques présentatrices de l'antigène. Enfin, ils peuvent jouer eux-mêmes le rôle d'effecteurs en produisant des lymphokines anti-virales comme l'IFN-γ et TNF-α.

Que le rôle bénéfique des lymphocytes T CD4+ dans la résolution de l'infection par le VHC résulte d'une action directe ou indirecte, il constitue un important argument en faveur de vaccins dirigés contre le VHC et capables de stimuler une forte réponse CD4+ de type Th1. En particulier, l'utilisation de peptides capables de stimuler ces cellules constituerait une approche vaccinale très intéressante à la fois efficace et économiquement viable. Toutefois, les peptides que reconnaissent les lymphocytes T auxiliaires sont difficiles à définir en raison du polymorphisme des molécules HLA II.

En effet, les lymphocytes T CD4+ ne reconnaissent les antigènes que sous la forme de peptides présentés par les molécules HLA II. Plus précisément, l'activation des lymphocytes T CD4+ se fait sous l'effet de la présentation de peptides par les molécules HLA II que portent les cellules présentatrices (APC). Ces peptides, appelés épitopes T, résultent de la dégradation protéolytique des antigènes par l'APC. Ils ont des longueurs variables généralement de 13 à 25 acides aminés et possèdent une séquence qui les rend capables de se lier aux molécules HLA II. Les molécules HLA II sont des hétérodimères, capables de lier un répertoire important de peptides ayant des séquences très différentes. Il existe quatre types de molécules HLA II par individu (2 HLA-DR, 1 HLA-DQ et 1 HLA-DP), la molécule HLA-DR dont la chaîne β est codée par le gène DRB1 étant la plus exprimée. Ces isoformes possèdent des propriétés de liaison différentes entre elles, ce qui implique qu'elles peuvent lier des peptides différents d'un même antigène. Elles sont très polymorphes et on a dénombré au moins 200 allèles différents pour DRB1. Les molécules issues des locus DRB3, DRB4, DRB5 et DPB1 sont moins polymorphes. Du fait de ce polymorphisme, chaque individu reconnaît dans un antigène un ensemble de peptides dont la nature dépend des molécules HLA II qui le caractérisent.

Au polymorphisme des molécules HLA II, il faut ajouter la difficulté de trouver des séquences conservées entre les différentes souches de VHC.

Un des moyens le plus utilisé pour définir les épitopes T CD4+ de type auxiliaire est de mesurer la capacité de peptides à faire proliférer les cellules mononucléées d'individus ayant été en contact avec l'antigène considéré.

Un certain nombre d'Auteurs ont identifié des peptides du VHC comme étant des épitopes T chez les patients étudiés :
- Diepolder et al., précités, ont montré l'existence d'épitopes du VHC issus de la protéine NS3 (NS3 1248-1261, NS3 1388-1407, NS3 1450-1469) reconnus par des lignées T auxiliaires obtenus à partir de patients en phase aiguë.
- Lamonaca et al. (Hepatology, 1999, 30, 10888) ont identifié cinq épitopes majeurs (Core 21-40, NS3 1253-1272, NS4 1767-1786, NS4 1907-1926, NS4 1909-1929), selon une approche similaire. Ces peptides sont à la fois immunodominants, conservés et présentés par plusieurs molécules HLA II ; sont plus particulièrement décrits 4 peptides immunodominants : C 21-40, NS3 1253-1272 et NS4 1767-1786, 1907-1926 et 1909-1929. Les tests de liaison à des molécules HLA II les plus fréquentes dans la population caucasienne et mondiale (DR1, DR4, DR7, DR11, DR15, DRB5, DR6, DR8 et DR9) présentés dans le tableau 3 de ce document montrent que seuls les peptides issus de NS3 et de NS4 (NS3 1253-1272, NS4 1767-1786 et NS4 1909-1929) se lient avec une activité de liaison inférieure à 1000 nM à aux moins 4 des molécules HLA II dont la fréquence est supérieure à 5 % dans la population caucasienne (DR1, DR4, DR11, DR7, DR15 et DRB5). En revanche, le peptide issu de la protéine C (C 21-40) se lie avec une forte affinité uniquement à une de ces molécules HLA II, à savoir DR15.
- Tabatabai et al. (Hum. Immunol. 1999, 60, 105) ont identifié plusieurs épitopes majeurs (NS3 1384-1401, NS3 1454-1471) chez un unique patient chroniquement infecté ; ces épitopes sont capables d'induire la prolifération et la production d'IL-2.
- Godkin et al. (Eur. J. Immunol., 2001, 31, 1438 ; voir également la Demande Internationale PCT WO 02/34770 au nom de Imperial College Innovations Ltd) ont identifié plusieurs épitopes T helper restreints par l'allèle HLA-DR11, chez plusieurs patients non-virémiques ou chroniquement infectés ; il s'agit de peptides et polypeptides restreints à DR11 et non de mélanges de peptides se liant aux molécules HLA II les plus fréquentes dans la population caucasienne.
- Hoffmann et al. (Hepatology, 1995, 21, 632) ont démontré que les cellules mononucléées du sang périphérique de patients en phase chronique reconnaissaient plusieurs peptides dérivés de la protéine Core.

Il existe d'autres études similaires, toutes basées sur les réponses de cellules mononucléées de patients infectés par le VHC (Woitas et al., J. Immunol., 1997, 159, 1012 ; Lechmann et al. , Hepatology, 1996, 24, 790 ; Leroux-Roels et al., Hepatology, 1996, 23, 8 ; Lohr et al., Liver, 1996, 16, 174.

Les peptides épitopes T identifiés dans ces études, qui sont issus des protéines les plus conservées du VHC (C et NS3), sont illustrés au Tableau I.

**Tableau I : Séquences des peptides C et NS3 identifiés comme étant des épitopes T par des tests de prolifération**

| **Peptide** | **Restriction HLA** | **Référence** | **Peptide** | **Restriction HLA** | **Référence** |
|---|---|---|---|---|---|
| **C1-24** | - | Woitas | **C131-150** | - | Hoffmann |
| **C21-40** | **DRB1*1101 DQB1*0301** | Lamonaca | **C133-152** | - | Leroux-Roels |
| **C20-44** | | Woitas Lechmann. | **C138-162** | | Lechmann |
| **C23-42** | **-** | Hoffmann | **C141-155** | **DR11** | Godkin WO 02/34770 |
| **C31-45** | **DR11** | Godkin WO 02/34770 | **C142-161** | - | Hoffmann |
| **C39-63** | **DR4** | Lechmann | **C141-160** | - | Woitas |
| **C41-60** | **-** | Lamonaca | **C145-164** | - | Leroux-Roels |
| **C47-70** | **-** | Lohr | **C148-172** | **DR11** | Woitas Lechmann |
| **C55-74** | **-** | Hoffmann | **C153-172** | - | Hoffmann |
| **C66-85** | **-** | Hoffmann | **C157-176** | - | Leroux-Roels |
| **C73-92** | **-** | Leroux-Roels | **C161-180** | **DRB1*08032** | Kaneko |
| **C71-90** | **-** | Lamonaca | **C168-192** | - | Woitas Lechmann |
| **C79-103** | **-** | Woitas Lechmann | **N1205-1221** | **DR11** | Godkin WO 02/34770 |
| **C81-100** | **-** | Lamonaca | **N1242-1261°** | | Diepolder |
| **C85-104** | **-** | Leroux-Roels | **N1248-1261** | **DRB1*0404** | Diepolder |
| | | | | **DRB1*1101** | |
| | | | | **DRB1*1201** | |
| | | | | **DRB1*1301** | |
| | | | | **DRB1*1601** | |
| **C91-110** | **-** | Lamonaca | **N1248-1267** | | Diepolder |
| **C97-116** | **-** | Leroux-Roels Lamonaca | **N1245-1269** | **DR11** | Godkin |
| **C101-115** | **-** | Lohr | **N1253-1272** | **DQB1*0301** | Lamonaca |
| **C111-130** | **DRB1*08032** | Kaneko | **N1293-1331** | - | Tabatai |
| **C109-128** | **-** | Leroux-Roels Hoffmann | **N1388-1407** | **DRB1*1501** | Diepolder |
| **C120-139** | **-** | Hoffmann | **N1384-1401** | - | Tabatai |
| **C121-140** | **-** | Leroux-Roels | **N1450-1469** | **DRB1*1302** | Diepolder |
| **C118-142** | | Woitas | **N1447-1464** | - | Tabatai |
| **C128-152** | **-** | Woitas Lechmann | **N1454-1471** | - | Tabatai |

| | | | | | |
|---|---|---|---|---|---|
| - Non déterminé / et variants de ces peptides dont le peptide minimum N1251-1259. | | | | | |

Il ressort du Tableau I que les séquences des épitopes T varient d'une étude à l'autre, traduisant le manque de précision de cette approche. Il est en effet difficile, au vu de la diversité des réponses observées, de définir des séquences épitopes T pour l'ensemble de la population caucasienne. Ces séquences ne sont adaptées qu'aux patients ayant servi à ces travaux. Ces différences de réponse s'expliquent d'une part par la représentativité des échantillons qui n'est pas évaluée. En particulier, dans les études où les patients ne sont pas typés pour leurs molécules HLA, nul ne sait si les différents allèles sont représentés selon les fréquences de la population générale. La réponse de nombreux patients à un peptide particulier peut alors résulter d'un biais de l'échantillonnage et non de la capacité effective d'un peptide à être reconnu par l'ensemble des patients. D'autre part, dans le cas de l'infection par le VHC, plusieurs études ont été effectuées sur des patients infectés de manière chronique. Du fait de la persistance du virus chez ces sujets, il est possible qu'ils présentent une tolérance aux épitopes T les plus efficaces à être présentés. Dans ce contexte, les épitopes les plus intéressants pourraient avoir disparu de la réponse immunitaire qui se maintiendrait pour des épitopes moins stimulants mais qui ne parviendrait pas à éliminer le virus. Cette hypothèse est d'ailleurs émise par Tabatabai et al., précité. Ces tests de prolifération sont donc insuffisants pour définir des séquences adaptées à la vaccination de l'ensemble de la population.

Parmi les peptides ayant une activité de stimulation de lymphocytes T identifiés, quelques uns seulement se lient aux molécules HLA II (Tableau II).

**Tableau II : Liaison des peptides C et NS3 aux molécules HLA II**

| **Peptides** | **Liaison à** |
|---|---|
| **C21-40** | DRB1*1501 |
| **N1242-1261** | DRB1*0101, 1501, 0401, 0404, 1101, 1302, 0701, 0802, 0901, DRB5*0101 |
| **N1248-1267** | DRB1*0101, 1501, 0401, 0404 1101, 1302, 0701, 0802, 0901 |
| **N1248-1261** | DRB1*0101, 1501, 0401, 0404, 0405,1101,1302,0802,0901 |
| **N1253-1272** | DRB1*0101, 0401, 1101, 701,1501, 1302, 0802, 0901 |
| **N 1388-1407** | AUCUN |
| **N1450-1469** | AUCUN |

Dans l'article aux noms de Diepolder et al., précité, les Auteurs ont en effet étudié pour 5 peptides de la protéine NS3, la capacité à lier des molécules HLA II fréquemment rencontrées. De fait, ils ont montré que l'épitope NS3 1248-1261 ou des peptides variants (1242-1261 et 1248-1267) présentent une capacité de liaison pour 9 à 10 allèles HLA-DR à savoir : DRB1*0101, 1501, 0401, 0404, 0405, 1101, 1302, 0802, 0901 et DRB5*0101. En revanche, les peptides 1388-1407 et 1450-1469 sont inactifs.

Dans l'article de Lamonaca et al., précité, les Auteurs ont également montré, par des tests de liaison, que le peptide Core 21-40 se lie à DRB1*1501 et que le peptide NS3 1253-1272 se lie à DRB1*0101, 0401, 1101, 701, 1501, 1302, 0802, 0901.

Il ressort de ce qui précède qu'aucun peptide du VHC se liant aux molécules HLA II les plus fréquemment rencontrées dans la population caucasienne, n'a été identifié pour la protéine C qui est la plus conservée et donc particulièrement adaptée à la vaccination contre les différents génotypes du VHC. En outre, parmi les peptides de la protéine NS3 ayant une activité de stimulation de lymphocytes T qui ont été étudiés, un seul peptide se lie aux molécules HLA II les plus fréquemment rencontrées dans la population caucasienne.

Il existe un nombre important de molécules HLA II dont la répartition n'est pas homogène dans le monde. Ainsi, dans une population donnée un ensemble d'allèles regroupe à lui seul l'essentiel des allèles de la population. Par exemple en France qui est une population caractéristique de la population caucasienne (USA, Europe), seuls 7 allèles du locus DRB1 dépassent les 5 %. Il s'agit des allèles DRB1*0101, DRB1*0301, DRB1*0401, DRB1*0701, DRB1*1101, DRB1*1301 et DRB1*1501 qui représentent à eux seuls 64% de la population. Ces mêmes allèles sont les plus abondants allèles HLA-DR dans les autres populations caucasiennes. Leur fréquence varie entre 53% (en Espagne) à 82% (au Danemark). Pour les Etats-Unis et le Canada, ils représentent respectivement 58 et 55% des allèles de la population. Les molécules HLA-DRB3, -DRB4 et DRB5 qui sont des molécules HLA-DR dont la chaîne β n'est pas codée par le gène DRB1 sont présentes également avec des fréquences alléliques importantes, car elles sont moins polymorphes que les molécules DRB1. Leur fréquence allélique est en effet de 9,2% pour DRB3*0101, 28,4% pour DRB4*0101 et 7,9% pour DRB5*0101. Elles couvrent donc à elles seules 45% de la fréquence allélique. Enfin, les molécules HLA-DP4 qui regroupent les molécules codées par les allèles DPB1*0401 et DPB1*0402 sont les molécules HLA II les plus abondantes en Europe et aux Etats-Unis. Leur fréquence allélique est en effet de 40 % et 11 % respectivement, ce qui signifie qu'elles sont l'une ou l'autre trouvées chez environ 76% des individus. Les peptides présents dans une séquence peptidique et qui lient l'ensemble de ces allèles inclus donc les épitopes T de la majorité de la population.

C'est pourquoi les Inventeurs se sont donnés pour but de pourvoir à un ensemble de peptides aptes à être incorporés dans une composition immunogène et à stimuler des lymphocytes T CD4+ anti-VHC, chez la majorité des individus caucasiens européens ou d'Amérique du Nord, pour induire effectivement une réponse proliférative et multiépitopique (qui fait appel à plusieurs épitopes) spécifique des composants du virus.

Un tel ensemble a pour propriété d'être efficace chez un grand nombre de sujets, alors que les peptides de l'art antérieur sont actifs chez quelques individus et sont inactifs chez la majorité des autres individus, parce que ces derniers ne reconnaissent pas les protéines du VHC par les mêmes déterminants.

Pour ce faire, les Inventeurs ont identifié les séquences de peptides issus des protéines C et NS3 du VHC restreints aux molécules HLA II prépondérantes dans les populations caucasiennes et ils ont montré que les peptides issus de la protéine C, de préférence associés à des peptides issus de la protéine NS3, induisent effectivement une réponse immunogène et protectrice chez un grand nombre d'individus, qui implique plusieurs épitopes.

En outre, ces peptides qui reconnaissent des lymphocytes T spécifiques d'HCV chez les patients infectés, sont utiles pour le diagnostic de l'état immunitaire de ces patients vis-à-vis du virus de l'hépatite C.

La présente invention a en conséquence pour objet un mélange de peptides, incluant au moins deux peptides différents issus du virus de l'hépatite C (VHC) pour utilisation comme vaccin contre l'infection par le virus de l'hépatite C, caractérisé en ce que l'un au moins des peptides est un peptide issu de la protéine C qui se lie avec une activité de liaison < 1000 nM, à au moins quatre molécules HLA Il différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4, lequel peptide étant sélectionné dans le groupe constitué par :
a) le peptide correspondant aux positions 127-167,
b) les peptides d'au moins 11 acides aminés comprenant les positions 134 à 145, inclus dans le peptide tel que défini en a), et
c) les peptides dérivés des peptides tels que définis en a) ou en b) par substitution par des résidus alanine (C → A), de résidu(s) cystéine situé(s) en position +1 ou +2, relativement au résidu d'acide aminé en position N terminale et/ou en position -1, -2 ou -3, relativement au résidu d'acide aminé en position C terminale.

Conformément à l'invention, ledit mélange comprend également, outre ledit peptide C tel que défini ci-dessus, un ou plusieurs peptides ou lipopeptides contenant un ou plusieurs épitopes CD8+, CD4+ ou B et plus particulièrement des épitopes issus d'une protéine du VHC, notamment au moins un peptide issu de la protéine NS3, se liant à au moins quatre molécules HLA II différentes dont la fréquence allélique est supérieure à 5 % dans la population caucasienne, avec une activité de liaison < 1000 nM.

L'invention englobe les peptides issus des protéines C et NS3 de n'importe quel génotype du VHC.

De manière particulièrement avantageuse, les dites molécules HLA II sont codées respectivement par les allèles HLA DRB1*0101, DRB1*0301, DRB1*0401, DRB1*0701, DRB1*1101, DRB1*1301, DRB1*1501, DRB3*0101, DRB4*0101, DRB5*0101, DP*0401 et DP*0402.

Un tel mélange de peptides permet d'obtenir, de manière surprenante, une réponse proliférative T CD4+ (stimulation des lymphocytes T CD4+) et ce, chez la grande majorité de la population caucasienne à protéger et quel que soit le génotype du VHC concerné ; on peut donc considérer qu'un tel mélange constitue un premier pas vers une composition immunogène « universelle », apte à être effectivement utilisée dans un vaccin.

Avantageusement, les peptides d'au moins 11 acides aminés tels que définis en b) sont sélectionnés dans le groupe constitué par : les peptides correspondant respectivement aux positions 127-149, 131-145, 131-148, 131-167 et 134-148.

Avantageusement, le peptide tel que défini en c) est le peptide de séquence TAGFADLMGYIPLVGAPLGGAAR (SEQ ID NO: 5), dérivé du peptide C 127-149.

Selon une autre disposition avantageuse dudit mélange, les peptides issus de la protéine NS3, sont sélectionnés dans le groupe constitué par :
d) les peptides correspondant respectivement aux positions 1007-1037, 1036-1055, 1052-1072, 1076-1093, 1127-1153, 1149-1172, 1174-1195, 1190-1212, 1206-1239, 1246-1275, 1275-1304, 1361-1387, 1377-1403, 1404-1432, 1456-1481, 1495-1513, 1524-1553 et 1552-1583,
e) les peptides d'au moins 11 acides aminés inclus dans les peptides précédents, et
f) les peptides dérivés des peptides tels que définis en d) ou en e) par substitution par des résidus alanine (C → A), de résidu(s) cystéine situé(s) en position +1 ou +2, relativement au résidu d'acide aminé en position N terminale et/ou en position-1, -2 ou -3, relativement au résidu d'acide aminé en position C terminale.

Avantageusement, les peptides d'au moins 11 acides aminés tels que définis en e) sont sélectionnés dans le groupe constitué par :
- les peptides inclus dans le peptide 1007-1037 correspondant respectivement aux positions 1007-1021, 1015-1029, 1015-1037, 1019-1033 et 1020-1034,
- les peptides inclus dans le peptide 1174-1195 correspondant respectivement aux positions 1174-1188, 1174-1192 et 1178-1192,
- les peptides inclus dans le peptide 1190-1212 correspondant respectivement aux positions 1190-1204 et 1192-1206,
- les peptides inclus dans le peptide 1246-1275 correspondant respectivement aux positions 1246-1260, 1246-1264, 1250-1264 et 1261-1275,
- les peptides inclus dans le peptide 1377-1403 correspondant respectivement aux positions 1381-1395, 1381-1397, 1381-1403 et 1383-1397,
- le peptide inclus dans le peptide 1495-1513 correspondant respectivement aux positions 1495-1509,
- les peptides inclus dans le peptide 1524-1553 correspondant respectivement aux positions 1524-1552, 1524-1538, 1528-1542, 1528-1552, 1529-1543, 1534-1548, 1538-1552 et 1540-1553, et
- les peptides inclus dans le peptide 1552-1583 correspondant respectivement aux positions 1559-1573 et 1563-1577.

Avantageusement, les peptides tels que définis en f) sont sélectionnés dans le groupe constitué par :
- le peptide dérivé du peptide 1076-1093 de séquence GVAWTVYHGAGTRTIASP (SEQ ID NO: 10),
- le peptide dérivé du peptide 1149-1172 de séquence RGSLLSPRPISYLKGSSGGPLLAP (SEQ ID NO: 13),
- le peptide dérivé du peptide 1377-1403 de séquence GKAIPLEVIKGGRHLIFCHSKKKADEL (SEQ ID NO: 20),
- le peptide dérivé du peptide 1456-1481 de séquence TAVTQTVDFSLDPTFTIETITLPQDA (SEQ ID NO: 22), et
- le peptide dérivé du peptide 1524-1553 de séquence GAAWYELTPAETTVRLRAYMNTPGLPVAQD (SEQ ID NO: 24) et les peptides inclus dans la séquence SEQ ID NO: 24 correspondant respectivement aux positions 1524-1538, 1524-1552, 1528-1552, 1538-1552 et 1540-1553.

Selon un autre mode de réalisation avantageux dudit mélange, il inclut des peptides issus des protéines C et NS3 du VHC de génotype 1, de préférence de sous-type 1a ou 1b.

Selon encore un autre mode de réalisation avantageux dudit mélange, il inclut 2 à 6 peptides différents issus des protéines C et NS3, tels que définis ci-dessus, l'ensemble des peptides se liant à au moins 10 molécules HLA II dont la fréquence allélique est supérieure à 5 % dans la population caucasienne.

Selon une disposition avantageuse de ce mode de réalisation, lesdits peptides sont sélectionnés dans le groupe constitué par les peptides issus de la protéine C correspondant respectivement aux positions 27-51, 131-167, 127-149, 131-148, 148-167 et les peptides issus de la protéine NS3 correspondant respectivement aux positions 1007-1037, 1015-1037, 1036-1055, 1174-1192, 1190-1212, 1246-1264, 1381-1403, 1381-1397, 1524-1553, 1528-1552 et 1552-1583.

Par exemple:
- le peptide C 19-47 se lie avec une bonne affinité aux molécules DR1, DR7, DR11, DR13, DR15, DRB5 et DP402 codées par les allèles tels que définis ci-dessus,
- le peptide C 31-57 se lie avec une bonne affinité aux molécules DR1, DR7, DR11, DR13, DR15, DRB5, DP401 et DP402 codées par les allèles tels que définis ci-dessus,
- le peptide C 104-133 se lie avec une bonne affinité aux molécules DR1, DR7, DR11 et DRB5 codées par les allèles tels que définis ci-dessus,
- le peptide C 127-149 se lie avec une bonne affinité aux molécules DR1, DR7, DR11, DR15 et DRB5 codées par les allèles tels que définis ci-dessus,
- le peptide NS3 1007-1037 se lie avec une bonne affinité aux molécules DR1, DR3, DR4, DR7, DR11, DR13, DR15, DRB4, DRB5 et DP402 codées par les allèles tels que définis ci-dessus,
- le peptide NS3 1036-1055 se lie avec une bonne affinité aux molécules DR1, DR4, DR11, DRB4 et DRB5, codées par les allèles tels que définis ci-dessus,
- le peptide NS3 1052-1080 se lie avec une bonne affinité aux molécules DR1, DR4, DR7, DR11, DR15, DRB4 et DRB5, codées par les allèles tels que définis ci-dessus,
- le peptide NS3 1076-1093 se lie avec une bonne affinité aux molécules DR1, DR7, DR11, DR15, et DRB5, codées par les allèles tels que définis ci-dessus,
- le peptide NS3 1127-1153 se lie avec une bonne affinité aux molécules DR1, DR7, DR11, DR13, et DRB5, codées par les allèles tels que définis ci-dessus,
- le peptide NS3 1149-1172 se lie avec une bonne affinité aux molécules DR1, DR7, DR15, DRB4, et DRB5, codées par les allèles tels que définis ci-dessus,
- le peptide NS3 1174-1195 se lie avec une bonne affinité aux molécules DR1, DR4, DR7, DR11, DR15, DRB4, et DRB5, codées par les allèles tels que définis ci-dessus
- le peptide NS3 1190-1212 se lie avec une bonne affinité aux molécules DR1, DR4, DR7, DR11, DR15 et DRB5, codées par les allèles tels que définis ci-dessus,
- le peptide NS3 1206-1239 se lie avec une bonne affinité aux molécules DR1, DR4, DR7, DR11 et DRB5, codées par les allèles tels que définis ci-dessus,
- le peptide NS3 1246-1275 se lie avec une bonne affinité aux molécules DR1, DR4, DR7, DR11, DR13 et DR15, codées par les allèles tels que définis ci-dessus,
- le peptide NS3 1275-1304 se lie avec une bonne affinité aux molécules DR1, DR4, DR7, DR11, DR15, DRB3 et DP401 codées par les allèles tels que définis ci-dessus,
- le peptide NS3 1361-1387 se lie avec une bonne affinité aux molécules DR1, DR7, DR15 et DRB5 codées par les allèles tels que définis ci-dessus,
- le peptide NS3 1377-1403 se lie avec une bonne affinité aux molécules DR1, DR7, DR11, DR13, DRB4 et DRB5 codées par les allèles tels que définis ci-dessus,
- le peptide NS3 1404-1432 se lie avec une bonne affinité aux molécules DR1, DR4, DR7, DR15 et DRB5 codées par les allèles tels que définis ci-dessus,
- le peptide NS3 1456-1481 se lie avec une bonne affinité aux molécules DR1, DR3, DR4, DR7, DR11, DR13, DR15, DRB3, DRB4 et DRB5 codées par les allèles tels que définis ci-dessus,
- le peptide NS3 1495-1513 se lie avec une bonne affinité aux molécules DR1, DR7, DR11, DR15, DRB3, DRB4 et DRB5 codées par les allèles tels que définis ci-dessus,
- le peptide NS3 1524-1553 se lie avec une bonne affinité aux molécules DR1, DR4, DR7, DR11, DR15, DRB3, DRB4, DRB5 et DP402 codées par les allèles tels que définis ci-dessus,
- le peptide NS3 1552-1583 se lie avec une bonne affinité aux molécules DR1, DR7, DR11, DR15, DRB5, DP401 et DP402 codées par les allèles tels que définis ci-dessus.

Conformément à l'invention, les peptides inclus dans ledit mélange sont sous forme, soit de peptides individualisés, soit d'une protéine de fusion comprenant une séquence des peptides dudit mélange.

Lesdits peptides individualisés sont préparés selon les procédés classiques de synthèse en parallèle sur phase solide et ladite protéine de fusion est préparée selon les techniques classiques d'ADN recombinant, dans un système d'expression approprié.

Ladite protéine de fusion comprend les séquences desdits peptides directement reliées entre-elles par une liaison peptidique ou bien séparées par des séquences exogènes, c'est-à-dire des séquences autres que celles présentes à cette position dans la séquence des protéines C et NS3 du VHC, notamment les séquences d'autres épitopes T CD4+, CD8+ ou B, par exemple du VHC.

La présente invention a également pour objet une molécule d'acide nucléique, caractérisée en ce qu'elle code pour une protéine de fusion telle que définie ci-dessus.

L'invention a également pour objet tout vecteur recombinant, notamment plasmide ou virus, comprenant au moins une molécule d'acide nucléique telle que définie ci-dessus, placée sous le contrôle des éléments nécessaires à la transcription de ladite molécule, notamment sous le contrôle d'un promoteur et d'un terminateur de transcription.

L'invention concerne également les cellules hôtes, notamment bactéries, levures ou cellules de mammifère, transformées à l'aide d'un vecteur tel que défini ci-dessus, de manière à intégrer de façon stable dans leur génome ou à maintenir de manière stable, au moins une molécule d'acide nucléique telle que définie ci-dessus.

La présente invention a également pour objet une composition immunogène anti-VHC, caractérisée en ce qu'elle comprend au moins :
- un mélange de peptides issus d'une protéine C et d'une protéine NS3 du VHC, tels que définis ci-dessus, et/ou
- une molécule d'acide nucléique telle que définie ci-dessus, ou
- un vecteur approprié tel que défini ci-dessus, notamment un virus, en association avec au moins un véhicule pharmaceutiquement acceptable et éventuellement au moins un adjuvant.

Les adjuvants utilisés sont des adjuvants classiquement utilisés dans les compositions vaccinales, tels que l'hydroxyde d'alumine et le squalène.

On peut utiliser entre autres des vecteurs viraux tels que les adéno-virus, les rétrovirus, les lentivirus et les AAV, dans lesquels a été insérée préalablement la séquence d'intérêt ; on peut également associer ladite séquence (isolée ou insérée dans un vecteur plasmidique) avec une substance capable d'assurer une protection desdites séquences dans l'organisme ou lui permettant de franchir la membrane des cellules-hôte, par exemple une préparation de liposomes, de lipides ou de polymères cationiques, ou bien l'injecter directement dans la cellule hôte, sous forme d'ADN nu.

Par exemple, l'utilisation d'ADN nu pour l'immunisation constitue une approche vaccinale efficace : elle consiste à injecter dans l'organisme hôte à vacciner, un ADN nu codant pour un antigène protéique ; cet ADN permet une synthèse prolongée de l'antigène par les cellules de l'hôte ainsi qu'une présentation durable de cet antigène au système immunitaire.

Selon un mode de réalisation avantageux de ladite composition immunogène, lesdits peptides sont sous forme de peptides modifiés ou bien associés à des liposomes ou à des lipides, notamment sous forme de lipopeptides.

La partie lipidique du lipopeptide est notamment obtenue par addition d'un motif lipidique sur une fonction α-aminée desdits peptides ou sur une fonction réactive de la chaîne latérale d'un acide aminé de la partie peptidique ; elle peut comprendre une ou plusieurs chaînes dérivées d'acides gras en C₄₋₂₀, éventuellement ramifiées ou insaturées (acide palmitique, acide oléique, acide linoléique, acide lino-lénique, acide 2-amino hexadécanoïque, pimélautide, trimétauxide) ou un dérivé d'un stéroïde. Le procédé de préparation de tels lipopeptides est notamment décrit dans les Demandes Internationales WO 99/40113 ou WO 99/51630. La partie lipidique préférée est notamment représentée par un groupe N^{α}-acétyl-lysine N^{ε}(palmitoyl), également dénommé Ac-K(Pam).

Le peptide modifié est notamment obtenu par modification d'au moins une liaison peptidique -CO-NH- de la chaîne peptidique desdits peptides par introduction d'une liaison de type rétro ou rétro-inverso (-NH-CO-) ou d'une liaison différente de la liaison peptidique (méthylène amino, carba, cétométhylène, méthylène-oxy...) ou bien par substitution d'au moins un acide aminé de la chaîne peptidique desdits peptides par un acide aminé non protéinogénique, c'est à dire un acide aminé n'entrant pas dans la constitution d'une protéine naturelle, notamment un acide aminé dont le carbone portant la chaîne latérale, à savoir le groupe -CHR- est remplacé par un motif n'entrant pas dans la constitution d'un acide aminé naturel.

Selon un autre mode de réalisation avantageux de ladite composition immunogène, ledit mélange de peptides est associé :
- à un ou plusieurs peptides ou lipopeptides contenant un ou plusieurs épitopes CD8+ (reconnus spécifiquement par les lymphocytes T cytotoxiques et présentés par les molécules HLA I) et plus particulièrement des épitopes CD8+ issus d'une protéine du VHC tels que les peptides C 2-10, 28-36, 35-44, 41-49, 42-50, 85-98, 88-97, 127-140, 131-140, 132-140, 167-176, 178-187, 181-190 ; les peptides E1 220-227, 233-242, 234-242, 363-371 ; les peptides E2 401-411, 460-469, 489-496, 569-578, 621-628, 725-733 ; les peptides NS2 826-838, 838-845 ; les peptides NS3 1073-1081, 1169-1177, 1287-1296, 1395-1403, 1406-1415 ; les peptides NS4A 1585-1593, 1666-1675 ; les peptides NS4B 1769-1777, 1789-1797, 1807-1816, 1851-1859 ; le peptide NS5A 2252-2260 et les peptides NS5B 2588-2596, 2727-2735 (Rehermann et al., Current Topics in Microbiology and Immunology, 2000, 242; 299),
- à d'autres peptides comprenant des épitopes CD4+ multiples, tels que le peptide de la toxine tétanique TT (positions 830-846), le peptide de l'hémagglutinine d*'Influenza* HA (positions 307-319), PADRE (Pan DR Epitope, Alexandre J. et al., Immunity, 1994,1,9, 751-761) et le peptide LSA3 de *Plasmodium falciparum* et/ou
- à un ou plusieurs peptides ou lipopeptides contenant un ou plusieurs épitopes B, plus particulièrement des épitopes B issus d'une protéine du VHC reconnus spécifiquement par des anticorps dirigés contre ces derniers, tels que le peptide C 5-27 (Khanna et al., Acta Virologica, 1998, 42, 141-145), le peptide NS4 1698-1719 (Khanna et al., précité) et le peptide NS5 2295-2315 (Khudyakov et al., Virology, 1995, 206, 666-672).

Les peptides C et NS3 selon l'invention, inclus dans les mélanges, tels que définis ci-dessus ont été avantageusement sélectionnés à l'aide d'un test de liaison HLA II/peptides comprenant :
- la purification des molécules HLA II d'intérêt, c'est-à-dire celles concernant plus de 5 % d'une population donnée et notamment les molécules HLA DR1, DR3, DR4, DR7, DR11, DR13, DR15, DRB3, DRB4, DRB5 et DP4,
- l'incubation des molécules HLA II ainsi purifiées, avec différentes concentrations de fragments chevauchants et couvrant la séquence de la protéine C ou de la protéine NS3 et avec un réactif R1 ou traceur constitué d'un fragment peptidique associé à un marqueur non radioactif, tel que la biotine et dont la séquence est différente desdits peptides ; le réactif R1 ou traceur est choisi de manière à ce qu'il présente une affinité vis-à-vis de l'une des molécules HLA II d'intérêt, telle qu'il puisse être utilisé à une concentration < 200 nM,
- le transfert des complexes obtenus sur une plaque de type ELISA, préalablement sensibilisée avec un anticorps spécifique de toutes les molécules DR ou DP,
- la révélation des complexes molécules HLA II/réactif R1, fixés au fond de la plaque au moyen de conjugués convenables, tels que streptavidine-phosphatase et d'un substrat fluorescent,
- la sélection des peptides comprenant des épitopes différents, c'est-à-dire les plus représentatifs des différentes zones d'interaction entre la protéine C ou la protéine NS3 et les molécules HLA II et
- le choix des peptides les plus adaptés, en fonction de la fréquence des allèles vis-à-vis desquels ils présentent une activité de liaison < 1000 nM, de préférence < 800 nM, correspondant à la concentration de ces peptides, qui inhibe 50 % de la liaison du réactif R1 (IC₅₀).

Ces tests permettent, de manière non ambiguë, d'associer à chaque molécule HLA II, les séquences des fragments capables de s'y lier ou au contraire qui ne s'y lient pas.

Cette démarche permet de définir des compositions immunogènes incluant des peptides qui se lient au plus grand nombre de molécules HLA II différentes et qui peuvent être ainsi avantageusement protectrices pour la majorité des patients, même si l'on ne connaît pas leurs génotype HLA.

Cette démarche a en outre l'avantage de permettre la sélection de peptides significativement plus spécifiques vis-à-vis du VHC que les démarches cherchant à sélectionner des peptides sur la base de leur capacité à stimuler les lymphocytes T CD4+ (tests de prolifération).

Les conditions d'incubation sont propres à chaque molécule HLA II (temps d'incubation, pH, réactif R1, concentration en HLA II ou en réactif R1).

Le réactif R1 est sélectionné dans le groupe constitué par les séquences suivantes :
- PKYVKQNTLKLAT (HA 306-318, SEQ IDNO: 75), spécifique des allèles DRB1*0101, DRB1*0401, DRB1*1101 et DRBS*0101,
- EAEQLRAYLDGTGVE (A3 152-166, SEQ ID N0: 79), spécifique de l'allèle DRB1*1501,
- AKTIAYDEEARGLE (MT 2-16, SEQ ID NO: 77), spécifique de l'allèle DRB1*0301,
- AAYAAAKAAALAA (YKL, SEQ ID NO: 76), spécifique de l'allèle DRB1*0701,
- TERVRLVTRHIYNREE (B1 21-36, SEQ ID NO: 78), spécifique de l'allèle DRB1*1301,
- ESWGAVWRIDTPDKLTGPFT (LOL 191-210, SEQ ID NO: 80), spécifique de l'allèle DRB3*0101,
- AGDLLAIETDKATI (E2/E168, SEQ ID NO: 81), spécifique de l'allèle DRB4*0101, et
- EKKYFAATQFEPLAARL (Oxy 271-287, SEQ ID NO: 82) spécifique des allèles DP*0401 et DP*0402.

D'autres réactifs R1 peuvent être utilisés, notamment ceux décrits dans Southwood et al. (J. Immunol., 1998, 160, 3363-3373).

La présente invention a également pour objet un vaccin destiné à la prévention et au traitement des infections à VHC, caractérisé en ce qu'il inclut une composition immunogène telle que définie ci-dessus.

La présente invention a également pour objet des peptides issus de la protéine C d'un VHC, notamment de génotype 1a ou 1b, caractérisés en ce qu'ils sont sélectionnés dans le groupe constitué par : les peptides issus de la protéine C, tels que définis ci-dessus, à l'exclusion des peptides C 131-150 et C 133-152.

De tels peptides qui contiennent un épitope CD4+, apte à avoir une activité de liaison < 1000 nM, de préférence < 800 nM vis-à-vis d'au moins 4 molécules HLA II différentes telles que définies ci-dessus, sont aptes à être reconnus par des lymphocytes T CD4+ spécifiques desdits peptides présents chez des patients infectés par le VHC sont donc utiles comme réactifs de diagnostic d'une infection à VHC.

La présente invention a également pour objet un réactif de diagnostic, caractérisé en ce qu'il comprend au moins un peptide C, tel que défini ci-dessus, ledit peptide étant éventuellement marqué ou complexé, sous la forme de complexes multimériques.

La présente invention a également pour objet un procédé *in vitro* d'évaluation de l'état immunitaire d'un individu, caractérisé en ce qu'il comprend une étape de détection de la présence de cellules T CD4+ spécifiques d'un peptide C, tel que défini ci-dessus ; ladite détection est réalisée, de manière avantageuse par l'un des tests suivants : test de prolifération, test ELISPOT [voir par exemple la Demande Internationale WO 99/51630 ou Gahéry-Ségard et al. J. Virol., 2000, 74, 1964-)] ou cytométrie de flux en présence de complexes multimériques constitués à partir desdits peptides E6 et/ou E7.

De manière plus précise :
* pour ce qui concerne le test de prolifération :
   Une suspension de cellules (PBMC, PBMC déplétées en cellules CD8+, lymphocytes T préalablement enrichis par une étape de culture *in vitro* avec les peptides sélectionnés selon l'invention ou des lymphocytes T clonés) est cultivée pendant 3 à 5 jours en présence des peptides sélectionnés et au besoin de cellules présentatrices appropriées telles que des cellules dendritiques, des PBMC autologues ou hétérologues, des cellules lymphoblastoïdes telles que celles obtenues après infection par le virus EBV ou des cellules génétiquement modifiées. La prolifération des cellules est mesurée par incorporation de thymidine tritiée dans l'ADN des cellules. Les peptides sélectionnés conformément à l'invention, permettent de révéler dans la suspension initiale la présence de cellules spécifiques de ces peptides.
* pour ce qui concerne le test ELISPOT :
   Le test ELISPOT permet de révéler la présence de cellules T spécifiques d'un peptide sélectionné conformément à l'invention et sécrétant de l'IFN-γ.
   De manière plus précise, les cellules T sont révélées par mesure de la sécrétion d'IFN-γ après incubation des PMBC des patients avec les peptides sélectionnés selon l'invention, conformément à la méthode décrite dans Gahéry-Ségard et al., J. Virol., 2000, 74, 1964.
* pour ce qui concerne la mise en oeuvre de complexes multimériques et notamment de complexes tétramériques :
   - on met en contact un échantillon biologique, de préférence des cellules mononucléées du sang périphérique (PBMC) avec des complexes tétramériques marqués produits à partir de complexes entre un peptide C tel que défini ci-dessus et des molécules HLA de classe II solubles et l'on
   - analyse les cellules marquées, notamment par cytométrie de flux.

De manière avantageuse, préalablement à la mise en contact de l'échantillon biologique avec ledit complexe, on l'enrichit en cellules T CD4+, en le mettant en contact avec des anticorps anti-CD4, pour enrichir ledit échantillon.

Les tétramères sont préparés, comme précisé, par exemple dans E.J. Novak et al. (J. Clin. Investig., 1999, 104, R63-R67) ou dans M.J. Kuroda et al. (J. Virol., 2000, 74, 18, 8751-8756).

Brièvement, les tétramères sont fabriqués en incubant, pendant 72 heures à 37°C et dans un tampon phosphate citrate 10 mM, NaCl 0,15 M à un pH compris entre 4,5 et 7, des molécules HLA II solubles et biotinylées avec un excès de 10 de peptides C et/ou NS3, identifiés et sélectionnés conformément à l'invention.

La forme tétramérisée est obtenue en ajoutant à la préparation de la streptavidine marquée par un fluorochrome en quantité quatre fois moindre (mole à mode) que de molécules HLA II. L'ensemble est incubé une nuit à température ambiante.

Pour utiliser ces tétramères, on met en contact une suspension de cellules (PBMC, PBMC déplétées en cellules CD8+, lymphocytes T préalablement enrichis par une étape de culture *in vitro* avec les peptides C sélectionnés conformément à la présente invention ou des lymphocytes T clonés) avec un ou plusieurs tétramères (10 à 20 mg/ml) pendant 1 à 3 heures. Après lavage, la suspension est analysée par cytométrie de flux : on visualise le marquage des cellules par les tétramères grâce au fait que ces constructions sont fluorescentes.

La cytométrie de flux permet de séparer les cellules marquées par les tétramères des cellules non marquées et d'effectuer ainsi un tri cellulaire.

La présente invention a ainsi, en outre, pour objet une méthode de tri de lymphocytes T spécifiques du VHC, caractérisé en ce qu'il comprend au moins les étapes suivantes :
- incubation ou mise en contact, d'une suspension de cellules à trier avec un ou plusieurs tétramères marqués formés à partir de complexes entre des peptides C et/ou NS3 tels que définis ci-dessus et des molécules HLA II solubles, et
- tri des cellules marquées par les tétramères.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'au dessins annexés, dans lesquels :

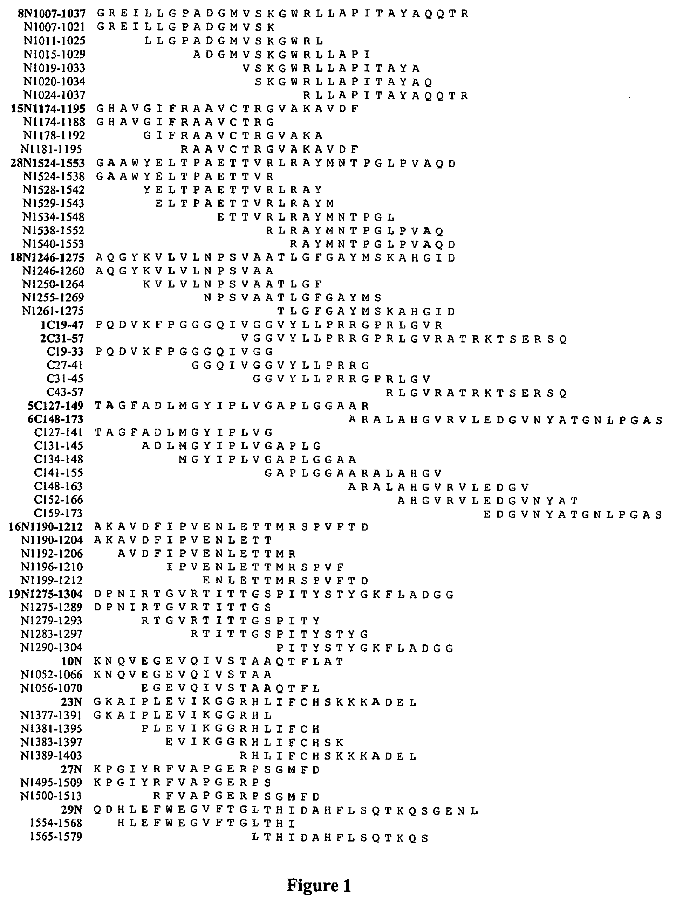

- la figure 1 illustre les séquences des peptides issus des protéines C et NS3 qui ont été étudiés. Pour simplifier les différents peptides ont été dénommés 1C à 6C et 8N à 29N.

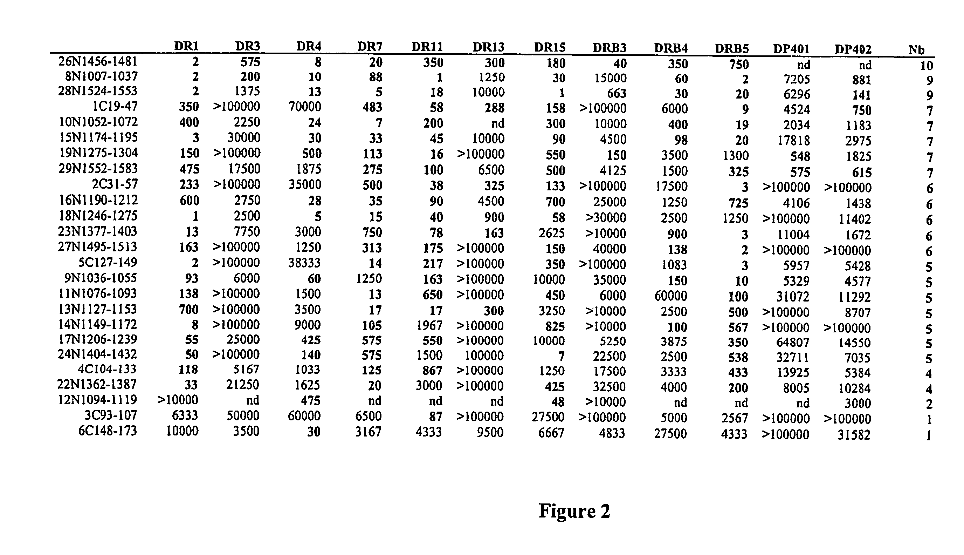

- la figure 2 illustre l'activité de liaison d'une première série de peptides des protéines C et NS3, vis-à vis des molécules HLA II prépondérantes dans la population caucasienne. Les valeurs correspondent aux IC₅₀, exprimées en nM. Les valeurs inférieures à 1000 nM, correspondant aux peptides présentant une bonne affinité pour les molécules HLA II, sont indiquées en gras. nd : non déterminé.

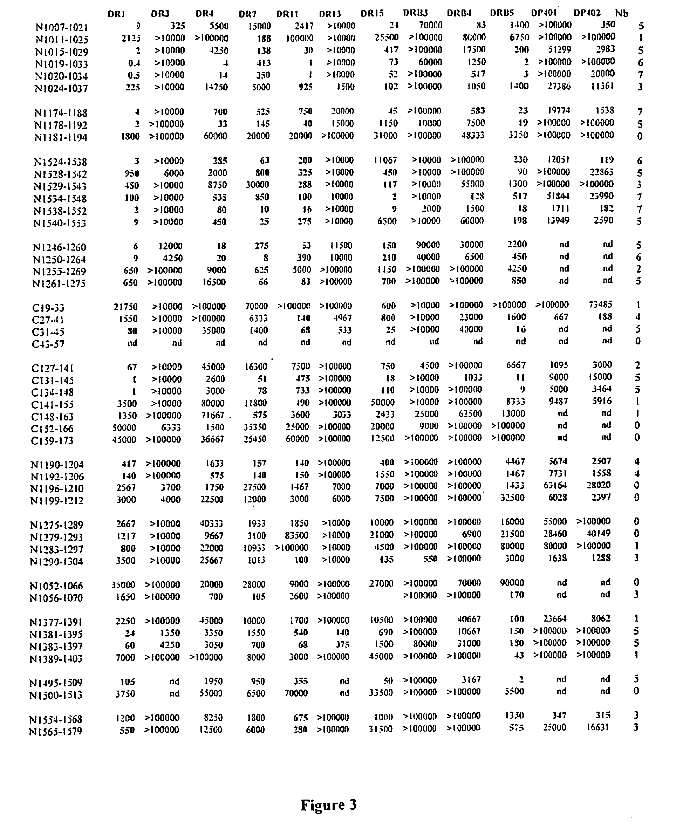

- la figure 3 illustre l'activité de liaison, vis-à vis des molécules HLA II prépondérantes dans la population caucasienne, d'une seconde série de peptide des protéines C et NS3, dérivés de la première série. Les valeurs correspondent aux IC₅₀, exprimées en nM. Les valeurs inférieures à 1000 nM, correspondant aux peptides présentant une bonne affinité pour les molécules HLA II, sont indiquées en gras. nd : non déterminé.

Il doit être bien entendu, toutefois, que ces exemples sont donnés uniquement à titre d'illustration de l'objet de l'invention, dont ils ne constituent en aucune manière une limitation.

### EXEMPLE 1: DETERMINATION DES CONDITIONS DES TESTS DE LIAISON PEPTIDES/MOLECULES HLA II

### 1) Peptides

Tous les peptides sont synthétisés selon la stratégie Fmoc en synthèse parallèle sur phase solide, purifiés par HPLC et contrôlés par spectrométrie de masse (ES-MS).

### a) Première série de peptides

L'activité de liaison des peptides des protéines C et NS3 du VHC aux molécules HLA II, a été testée à partir de 25 fragments de grandes tailles (entre 15 et 34 acides aminés), choisis selon deux critères :
- présence de plusieurs résidus aromatiques ou hydrophobes qui sont les principaux résidus d'ancrage pour les molécules HLA-DR et HLA-DP, et
- bonne probabilité de pouvoir être synthétisés.

Les séquences des peptides sélectionnés, issues du génotype 1 a, sont présentées dans le Tableau III ci-dessous.

**Tableau III : Séquences de la première série de peptides**

| **nom** | **Séquence** | **Taille** | **numéro** |
|---|---|---|---|
| 1C 19-47 | PQDVKFPGGGQIVGGVYLLPRRGPRLGVR | 29 | SEQ ID NO: 1 |
| 2C 31-57 | VGGVYLLPRRGPRLGVRATRKTSERSQ | 27 | SEQ ID NO: 2 |
| 3C 93-107 | WAGWLLSPRGSRPSW | 15 | SEQ ID NO: 3 |
| 4C 104-133 | RPSWGPTDPRRRSRNLGKVIDTLTCGFADL | 30 | SEQ ID NO: 4 |
| 5C 127-149 | TAGFADLMGYIPLVGAPLGGAAR | 23 | SEQ ID NO: 5 |
| 6C 148-173 | ARALAHGVRVLEDGVNYATGNLPGAS | 26 | SEQ ID NO: 6 |
| 8N 1007-1037 | GREILLGPADGMVSKGWRLLAPITAYAQQTR | 31 | SEQ ID NO: 7 |
| 9N 1036-1055 | TRGLLGCIITSLTGRDKNQV | 20 | SEQ ID NO: 8 |
| 10N 1052-1072 | KNQVEGEVQIVSTAAQTFLAT | 21 | SEQ ID NO: 9 |
| 11N 1076-1093 | GVAWTVYHGAGTRTIASP | 18 | SEQ ID NO: 10 |
| 12N 1094-1119 | KGPVIQMYTNVDQDLVGWPAPQGSRS | 26 | SEQ ID NO: 11 |
| 13N 1127-1153 | SSDLYLVTRHADVIPVRRRGDSRGSLL | 27 | SEQ ID NO: 12 |
| 14N 1149-1172 | RGSLLSPRPISYLKGSSGGPLLAP | 24 | SEQ ID NO: 13 |
| 15N 1174-1195 | GHAVGIFRAAVCTRGVAKAVDF | 22 | SEQ ID NO: 14 |
| 16N 1190-1212 | AKAVDFIPVENLETTMRSPVFTD | 23 | SEQ ID NO: 15 |
| 17N 1206-1239 | RSPVFTDNSSPPVVPQSFQVAHLHAPTGSGKSTK | 34 | SEQ ID NO: 16 |
| 18N 1246-1275 | AQGYKVLVLNPSVAATLGFGAYMSKAHGID | 30 | SEQ ID NO: 17 |
| 19N 1275-1304 | DPNIRTGVRTITTGSPITYSTYGKFLADGG | 30 | SEQ ID NO: 18 |
| 22N 1362-1387 | IEEVALSTTGEIPFYGKAIPLEVIKG | 26 | SEQ ID NO: 19 |
| 23N 1377-1403 | GKAIPLEVIKGGRHLIFCHSKKKADEL | 27 | SEQ ID NO: 20 |
| 24N 1404-1432 | AAKLVALGINAVAYYRGLDVSVIPTSGDV | 29 | SEQ ID NO: 21 |
| 26N 1456-1481 | TAVTQTVDFSLDPTFTIFTITLPQDA | 26 | SEQ ID NO: 22 |
| 27N 1495-1513 | KPGRRRFVAPGERPSGMFD | 19 | SEQ ID NO: 23 |
| 28N 1524-1553 | GAAWYELTPAETTVRLRAYMNTPGLPVAQD | 30 | SEQ ID NO: 24 |
| 29N 1552-1583 | QDHLEFWEGVFTGLTHIDAHFLSQTKQSGENL | 32 | SEQ ID NO: 25 |

| | | | |
|---|---|---|---|
| * les résidus alanine correspondant à la substitution d'un résidu cystéine de la séquence de la polyprotéine du VHC sont indiqués en gras. | | | |

### b) deuxième série de peptides

Tous les peptides de la première série se liant à au moins 6 molécules HLA II et certains peptides se liant à au moins 5 molécules HLA II ont été découpés en peptides de 15 acides aminés afin d'identifier plus précisément les zones d'interaction (peptides 8N, 9N, 10N, 15N, 16N, 18N, 19N, 23N, 27N, 28N, 29N, 1C, 2C et 5C). Seul le peptide 26 n'a pas été étudié en raison de la difficulté à le synthétiser. Le peptide 6C qui est très chevauchant avec le peptide 5C et qui en combinaison avec ce dernier se lie à 6 molécules HLA II a également été étudié.

La séquence de l'ensemble de ces peptides est présentée dans le Tableau IV et la figure 1.

**Tableau IV : Séquences de la deuxième série de peptides**

| **nom** | **Séquence** | **numéro** |
|---|---|---|
| 8N 1007-1037 | **GREILLGPADGMVSKGWRLLAPITAYAQQTR** | **SEQ ID NO: 7** |
| 8N 1007-1021 | GREILLGPADGMVSK | SEQ ID NO: 26 |
| 8N 101 11-1025 | LLGPADGMVSKGWRL | SEQ ID NO: 27 |
| 8N 1015-1029 | ADGMVSKGWRLLAPI | SEQ ID NO: 28 |
| 8N 1019-1033 | VSKGWRLLAPITAYA | SEQ ID NO: 29 |
| 8N 1020-1034 | SKGWRLLAPITAYAQ | SEQ ID NO: 30 |
| 8N 1024-1037 | RLLAPITAYAQQTR | SEQ ID NO: 31 |
| **15N 1174-1195** | **GHAVCIFRAAVCTRCVAKAVDF** | **SEQ ID NO: 14** |
| 15N 1174-1 188 | GHAVGIFRAAVCTRG | SEQ ID NO: 32 |
| 15N 1178-1192 | GIFRAAVCTRGVAKA | SEQ ID NO: 33 |
| 15N 1181-1195 | RAAVCTRGVAKAVDF | SEQ ID NO: 34 |
| **28N 1524-1553** | **GAAWYELTPAETTVRLRAYMNTPGLPVAQD** | **SEQ ID NO: 24** |
| 28N 1524-1538 | GAAWYELTPAETTVR | SEQ ID NO: 35 |
| 28N 1528-1542 | YELTPAETTVRLRAY | SEQ ID NO: 36 |
| 28N 1529-1543 | ELTPAETTVRLRAYM | SEQ ID NO: 37 |
| 28N 1534-1548 | ETTVRLRAYMNTPGL | SEQ ID NO: 38 |
| 28N 1538-1552 | RLRAYMNTPGLPVAQ | SEQ ID NO: 39 |
| 28N 1540-1553 | RAYMNTPGLPVAQD | SEQ ID NO: 40 |
| 18N 1246-1275 | AQGYKVLVLNPSVAATLGFGAYMSKAHGID | SEQ ID NO: 17 |
| 18N 1246-1260 | AQGYKVLVLNPSVAA | SEQ ID NO: 41 |
| 18N 1250-1264 | KVLVLNPSVAATLGF | SEQ ID NO: 42 |
| 18N 1255-1269 | NPSVAATLGFGAYMS | SEQ ID NO: 43 |
| 18N 1261-1275 | TLGFGAYMSKLAHGID | SEQ ID NO: 44 |
| **1C 19-47** | **PQDVKFPGGGQIVGGVYLLPRRGPRLGVR** | **SEQ ID NO: 1** |
| **2C 31-57** | **VGGVYLLPRRGPRLGVRATRKTSERSQ** | **SEQ ID NO: 2** |
| 1C 19-33 | PQDVKFPGGGQIVGG | SEQ ID NO: 45 |
| 1C 27-41 | GGGQIVGGVYLLPRRG | SEQ ID NO: 46 |
| 2C 31-45 | GGVYLLPRRGPRLGV | SEQ ID NO: 47 |
| 2C 43-57 | RLGVRATRKTSERSQ | SEQ ID NO: 48 |
| **5C 127-149** | **TAGFADLMGYIPLVGAPLGGAAR** | **SEQ ID NO: 5** |
| **6C 148-173** | **ARALAHGVRVLEDGVNYATGNLPGAS** | **SEQ ID NO: 6** |
| 5C 127-141 | TAGFADLMGYIPLVG | SEQ ID NO: 49 |
| 5C 131-145 | ADLMGYIPLVGAPLG | SEQ ID NO: 50 |
| 5C 134-148 | MGYIPLVGAPLGGAA | SEQ ID NO: 51 |
| C 141-155 | GAPLGGAARALAHG | SEQ ID NO: 52 |
| 6C 148-163 | ARALAHGVRVLEDGV | SEQ ID NO: 53 |
| 6C 152-166 | AHGVRVLEDGVNYAT | SEQ ID NO: 54 |
| 6C 159-173 | EDGVNYATGNLPGAS | SEQ ID NO: 55 |
| **16N 1190-1212** | **AKAVDFIPVENLETTMRSPVFTD** | **SEQ ID NO: 15** |
| 16N 1190-1204 | AKAVDFIPVENLETT | SEQ ID NO: 56 |
| 16N 1192-1206 | AVDFIPVENLETTMR | SEQ ID NO: 57 |
| 16N 1196-1210 | IPVENLETTMRSPVF | SEQ ID NO: 58 |
| 16N 1199-1212 | ENLETTMRSPVFTD | SEQ ID NO: 59 |
| **10N 1052-1072** | **KNQVEGEVQIVSTAAQTFLAT** | **SEQ ID NO: 9** |
| 10N 1052-1066 | KNQVEGEVQIVSTAA | SEQ ID NO: 60 |
| 10N 1056-1070 | EGEVQIVSTAAQTFL | SEQ ID NO: 61 |
| **23N 1377-1403** | **GKAIPLEVIKGGRHLIFCHSKKKADEL** | **SEQ ID NO: 20** |
| 23N 1377-1391 | GKAIPLEVIKGGRHL | SEQ ID NO: 62 |
| 23N 1381-1395 | PLEVIKGGRHLIFCH | SEQ ID NO: 63 |
| 23N 1383-1397 | EVIKGGRHLIFCHSK | SEQ ID NO: 64 |
| 23N 1389-1403 | RHLIFCHSKKKADEL | SEQ ID NO: 65 |
| 27N 1495-1513 | ICPGIYRFVAPGERPSGMFD | SEQ ID NO: 23 |
| 27N 1495-1509 | KPGIYRFVAPGERPS | SEQ ID NO: 66 |
| 27N 1500-1513 | RFVAPGERPSGMFD | SEQ ID NO: 67 |
| **29N 1552-1583** | **QDHLEFWEGVFTGLTHIDAHFLSQTKQSGENL** | **SEQ ID NO: 25** |
| 29N 1554-1568 | HLEFWEGVFTGLTHI | SEQ ID NO: 68 |
| 29N 1565-1579 | LTHIDAHFLSQTKQS | SEQ ID NO: 69 |
| 29N 1559-1573 | EGVFTGLTHIDAHFL | SEQ ID NO: 70 |
| 29N 1563-1577 | TGLTHIDAHFLSQTK | SEQ ID NO:71 |
| 29N 1569-1583 | DAHFLSQTKQSGENL | SEQ ID NO: 72 |
| **9N 1036-1055** | **TRGLLGCIITSLTGRDKNQV** | **SEQ ID NO: 8** |
| 9N 1036-1050 | RGLLGCIITSLTGRD | SEQ ID NO: 73 |
| 9N 1041-1055 | GCHTSLTGRDKNQV | SEQ ID NO: 74 |
| 19N **1275-1304** | **DPNIRTGVRTITTGSPITYSTYGKFLADGC** | **SEQ ID NO: 18** |
| 19N 1275-1289 | DPMRTGVRTITTGS | SEQ ID NO: 83 |
| 19N 1279-1293 | RTGVRTITTGSPITY | SEQ ID NO: 84 |
| 19N 1283-1297 | RTITTGSPITYSTYG | SEQ ID NO: 85 |
| 19N 1290-1304 | PITYSTYGKFLADGG | SEQ ID NO: 86 |

### 2) Molécules HLA II

### a) Choix des allèles

12 molécules HLA II (10 molécules HLA-DR et 2 molécules HLA-DP) les plus abondantes dans la population française et dont les fréquences alléliques sont caractéristiques de la population caucasienne, ont été sélectionnées (Tableau VIII):

### - molécules HLA-DR dont la chaîne β est codée par le gène DR1

Il s'agit des molécules HLA-DR1, -DR3, -DR4, -DR7, -DR11,-DR13 et -DR15 dont la chaîne β est codée par les allèles du locus DRB1 dont la fréquence dépasse les 5 % dans la population française : DRBI*0101, DRB1*0301, DRB1*0401, DRB1*0701, DRB1*1101, DRB1*1301 et DRB1*1501 qui représentent à eux seuls 64 % de la population. Ces mêmes allèles sont les allèles HLA-DR les plus abondants dans les autres populations caucasiennes. Leur fréquence varie entre 53 % (en Espagne) et 82 % (au Danemark). Pour les Etats-Unis et le Canada, ils représentent respectivement 58 et 55 % des allèles DR de la population.

### - molécules HLA-DR dont la chaîne β n'est pas codée par le gène DR1

Il s'agit des molécules HLA-DRB3, -DRB4 et -DRB5 dont la chaîne β est codée par les allèles les plus fréquents dans la population française : HLA-DRB3*0101 (9,2 %), HLA-DRB4*0101 (28,4 %), et HLA-DRB5*0101 (7,9 %). Ces molécules couvrent à elles seules 45 % de la fréquence allélique.

### - molécules HLA-DP

Il s'agit des molécules HLA-DP4 qui regroupent les molécules codées par les allèles DPB1*0401 et DPB1*0402. Ces molécules DP4 sont les molécules HLA II les plus abondantes en Europe et aux Etats Unis. Leur fréquence allélique est en effet de 40 et 11 % respectivement ce qui signifie qu'elles sont l'une ou l'autre trouvées chez environ 76% des individus.
Les peptides présents dans une séquence protéique et qui lient l'ensemble de ces molécules incluent donc les épitopes T CD4⁺de la majorité de la population.

### b) purification des molécules HLA II

Les molécules HLA II sont purifiées par immunoaffinité à partir de différentes lignées homozygotes de lymphocytes B humains transformées par le virus Epstein Barr (EBV).

L'origine des lignées EBV et les différents allèles qui les caractérisent sont décrits dans les Tableaux V et VI ci-dessous.

**Tableau V : Spécificité DR des lignées EBV**

| **Lignées** | **Spécificité** | **Allèle DRB1** | **Autres Allèles DRB** |
|---|---|---|---|
| LG2* | DR1 | DRB1*0101 | |
| HOM2 | DR1 | DRB1*0101 | |
| SCHU | DR15 | DRB1*1501 | DRB5*0101 |
| MAT* | DR3 | DRB1*0301 | DRB3*0101 |
| STEILIN | DR3 | DRB1*0301 | DRB3*0101 |
| BOLETH ° | DR4 | DRB1*0401 | DRB4*0101 |
| PREISS° | DR4 | DRB1*0441 | DRB4*0101 |
| PITOUT | DR7 | DRB1*0701 | DRB4*0101 |
| SWEIG | DR11 | DRB1*1101 | DRB3*0202 |
| HHKB°° | DR13 | DRB1*1301 | DRB3*0101 |

| | | | |
|---|---|---|---|
| ° Strang et al. J. Gen Virol., 1990, 71, 423, °° Tsukui et al., Cancer Res., 1996, 56, 3967 | | | |

**Tableau VI : Lignées EBV exprimant DP4**

| **Lignées** | **Spécificité DP** | **Allèle DPA1** | **Allèle DPB1** | **référence** |
|---|---|---|---|---|
| HOM2 | DP4 | DPA1*0103 | DPB1*0401 | ° |
| BOLETH | DP4 | DPA1*0103 | DPB1*0401 | ° |
| PITOUT | DP4 | DPA1*0103 | DPB1*0401 | SOUTHWOOD et al., J. Immunol., 1998, 160, 3363-3373 |
| HHKB | DP4 | DPA1*0103 | DPB1*0401 | DAVENPORTH et al, P. N.A.S., 1995, 92, 6567. |
| SHU | DP4 | DPA1*0103 | DPB1*0402 | ° |
| MLF | DP4 | DPA1*0103 | DPB1*0402 | ° |
| BM92 | DP4 | DPA1*0103 | DPB1*0402 | ° |

| | | | | |
|---|---|---|---|---|
| ° l'origine des lignées est décrite sur le site internet de la Collection européenne de Culture Cellulaire (http: // fuseiv.co.uk/camr/.) | | | | |

Les molécules HLA-DR et HLA-DP sont immunopurifiés au moyen des anticorps monoclonaux respectivement L243 (Smith et al., P.N.A.S., 1982, 79, 608-612) et B7/21 (WATSON et al., Nature, 1983, 304, 358-361), selon les protocoles décrits dans Texier et al.(J. Immunol., 2000, 164, 3177 ; Eur. J. Immunol., 2001, 31, 1837).

### 3) Tests de liaison HLA II/peptides

### a) principe des tests

Les tests de liaison des peptides aux molécules HLA-DP et HLA-DR sont des tests en compétition avec une révélation immuno-enzymatique, dérivés de ceux mis au point pour des molécules HLA-DR (HLA-DR1: MARSHALL et al., J. Immunol., 1994, 152, 4946- ; HLA-DR1, -DR2, -DR3, -DR4, -DR7, -DR11 et -DR13 : Demande de Brevet FR 99 0879 et TEXIER et al., précités).

Les tests de liaison sont réalisés de la manière suivante: les molécules HLA-DR ou HLA-DP sont diluées dans des plaques à 96 puits en polypropylène, dans du tampon phosphate 10mM, NaCl 150 mM, dodécyl maltoside (DM) 1 mM, citrate 10 mM, thimérosal 0,003%, à un pH et une dilution appropriés pour chaque molécule. Un peptide traceur biotinylé (Tableau VIII) est ajouté à une concentration donnée, ainsi que plusieurs concentrations de peptides à tester (peptide compétiteur). A la fin de l'incubation à 37°C (entre 24 et 72 heures selon les molécules), les échantillons sont neutralisés avec 50 µL de tampon Tris HCl 450 mM, pH 7,5, thimérosal 0,003 %, BSA 0,3 %, DM 1 mM. Ils sont ensuite transférés sur des plaques ELISA maxisorp (96 puits) sur lesquels les anticorps anti-DP ou anti-DR ont été préalablement adsorbés. L'incubation des échantillons sur ces plaques est réalisée pendant deux heures à température ambiante. Des lavages sont effectués entre chaque étape en tampon Tris HCl 0,1M, pH 7,5, Tween-20 0,05%. Le peptide biotinylé lié aux molécules HLA II est détecté par l'addition de 100 µL/puits du conjugué streptavidine-phosphatase alcaline (45 minutes) dilué au 1/2000 dans le tampon Tris 10mM, pH 7, NaCl 0,15M, Tween 20 0,05%, BSA 0,2%, thimérosal 0,003%, puis de 200 µL/puits du substrat 4-méthyl-umbelliféryl-phosphate (MUP) à la concentration de 100 µM, en tampon NaHCO₃ 0,05M, pH 9,8, MgCl₂ 1mM. L'émission de fluorescence par le produit de la réaction enzymatique est mesurée à 450 nm après excitation à 365 nm. La liaison maximale est déterminée en incubant le peptide traceur biotinylé avec la molécule HLA II en l'absence de peptide compétiteur. La spécificité de liaison est contrôlée par l'addition d'un excès de peptide non biotinylé. Le bruit de fond obtenu ne diffère pas significativement de celui obtenu en incubant le peptide biotinylé sans les molécules HLA II. Les résultats sont exprimés sous la forme de la concentration de peptide compétiteur qui inhibe 50% de la liaison maximale du peptide traceur biotinylé (IC₅₀).

### b) Conditions et sensibilité des tests

Pour chaque test de liaison, la concentration en molécules HLA II, la concentration du peptide traceur, le pH d'incubation et le temps d'incubation ont été optimisés comme précisé dans le Tableau VII ci-après.

**TABLEAU VII : Conditions des tests de liaison aux molécules HLA II**

| Allèles | dilution | Traceurs | Concentration traceur (nM) | pH optimal | Temps d'incubation (h) |
|---|---|---|---|---|---|
| DRB1*0101 | 1/40 à 1/400 | HA 306-318 | 1 | 6 | 24 |
| DRB1*0301 | 1/10 à 1/40 | MT 2-16 | 200 | 4,5 | 72 |
| DRB1*0401 | 1/20à 1/100 | HA 306-318 | 30 | 6 | 24 |
| DRB1*0701 | 1/20 à 1/100 | YKL | 10 | 5 | 24 |
| DRB1*1101 | 1/20 à 1/100 | HA 306-318 | 20 | 5 | 24 |
| DRB1*1301 | 1/10 à 1/40 | B121-36 | 200 | 4,5 | 72 |
| DRB1*1501 | 1/10 à 1/100 | A3 152-166 | 10 | 4,5 | 72 |
| DRB5*0101 | 1/10 à 1/100 | HA 306-318 | 10 | 5,5 | 24 |
| DRB3*0101 | 1/10 à 1/100 | Lol 191-120 | 10 | 5,5 | 24 |
| DRB4*0101 | 1/10 à 1/100 | E2/E168 | 10 | 5 | 72 |
| DBP1*401 | 1/20 à 1/400 | Oxy 271-287 | 10 | | 24 |
| DPB1*402 | 1/20 à 1/100 | Oxy 271-287 | 10 | | 24 |

La sensibilité de chaque test est reflétée par les IC₅₀ observées avec les peptides non-biotinylés qui correspondent aux traceurs et les résultats obtenus sont illustrés au Tableau VIII ci-après.

**Tableau VIII : Sensibilité des tests de liaison au molécules HLA II prépondérantes dans la population caucasienne**

| **Allèles** | **Fréquence** | **Peptides** | **Séquences** | **IC₅₀** |
|---|---|---|---|---|
| | | | **(SEQ ID NO: 75 à 82)** | **(nM)** |
| DRB1*0101 | 9,3 | HA 306-318 | PKYVKQNTLKLAT | 31 |
| DRB1 *0401 | 5,6 | HA 306-318 | PKYVKQNTLKLAT | 44 |
| DRB1*1101 | 9,2 | HA 306-318 | PKYVKQNTLKLAT | 38 |
| DRB1*0701 | 14,0 | YKL | AAYAAAKAAALAA | 34 |
| DRB1*0301 | 10,9 | MT 2-16 | AKTIAYDEEARRGLE | 100 |
| DRB1*1301 | 6,0 | B1 21-36 | TERVRLVTRHIYNREE | 330 |
| DRB1*1501 | 8,0 | A3 152-166 | EAEQLRRAYLDGTGVE | 14 |
| DRB5*0101 | 7,9 | HA 306-3 18 | PKWKQNTLKLAT | 6,5 |
| DRB3*0101 | 9,2 | Lot 191-120 | ESWGAVWRIDTPDKLTGPFT | 5 |
| DRB4*0101 | 28,4 | E2/E168 | AGDLLAIETDKATI | 2 |
| DPB1*401 | 40 | Oxy 271-287 | EKKYFAATQFEPLAARL | 10 |
| DPB1*402 | 11 | Oxy 271-287 | EKKYFAATQFEPLAARL | 10 |

| | | | | |
|---|---|---|---|---|
| Les fréquences indiquées, issues de Colombani (HLA: fonctions immunitaires et applications médicales. 1993. Eds John Libbey Eurotext).qui sont les fréquences alléliques en France, sont représentatives de celles de la population caucasienne. | | | | |

### EXEMPLE 2 : ACTIVITES DE LIAISON DES PEPTIDES C et NS3 VIS-A-VIS DES MOLECULES HLA II PREPONDERANTES DANS LA POPULATION CAUCASIENNE.

### 1) Première série de peptides (figure 2)

L'activité de liaison des peptides longs tels que définis à l'exemple 1, vis à vis des 12 molécules HLA II prépondérantes dans la population caucasienne (HLA-DR1, -DR3, -DR4, -DR7, -DR11, -DR13, -DR15, -DRB3, -DRB4, -DRB5, - DP401, et -DP402 ; tableau VIII) a été mesurée dans les conditions précisées à l'exemple 1.

Les résultats illustrés par la figure 2 montrent que chaque peptide se lie avec une bonne affinité (IC₅₀ < 1000 nM) à au moins une molécule HLA II et que plusieurs peptides se lient avec une bonne affinité à plusieurs molécules HLA II. Parmi ces derniers, les peptides 26N, 8N, 28N, 1C, 10N, 15N, 19N, 29N, 2C, 16N, 18N, 23N, 27N, 5C, 9N, 11N, 13N, 14N, 17N, 24N, 4C et 22N se lient avec une bonne affinité à au moins 4 molécules HLA II différentes.

### 2) Deuxième série de peptides (figures 3 et Tableau IX)

Les résultats illustrés par la figure 3 et le Tableau IX permettent de préciser les zones d'interaction des peptides avec les différentes molécules HLA II.

**Tableau IX: Activités de liaison des peptides 9N et 29N vis-à-vis des molécules HLA II prépondérantes dans la population caucasienne.**

| | DR1 | DR3 | DR4 | DR7 | DR11 | DR13 | DR15 | DRB3 | DRB4 | DRB5 |
|---|---|---|---|---|---|---|---|---|---|---|
| 9N 1036-1055 | 51 | >100000 | 950 | 2600 | 700 | >100000 | 5500 | | 1000 | 53 |
| 9N 1036-1050 | 16 | >100000 | 350 | 2500 | 1100 | >100000 | 1550 | | 300 | 2000 |
| 9N 10411055 | 190 | 62500 | 2900 | 25000 | 2600 | >100000 | 525 | | 65000 | 11 |
| 29N 1552-1583 | 170 | 6750 | 4000 | 750 | 90 | 27333 | 60 | | 25000 | 333 |
| 29N 1559-1573 | 29 | >100000 | 3000 | 18 | 7 | >100000 | 305 | | >100000 | 32 |
| 29N 1563-1577 | 8 | 3900 | 2100 | 16 | 817 | 3250 | 13 | | 40000 | 12 |
| 29N 1569-1583 | 950 | >100000 | 10500 | 3000 | 22 | >100000 | 2800 | | >100000 | 21000 |

### EXEMPLE 3 : DETECTION DE LYMPHOCYTES T SPECIFIQUES DU VHC A L'AIDE DES PEPTIDES C ET NS3.

La capacité des peptides possédant une affinité élevée pour les molécules HLA de classe II tels que définis ci-dessus, à détecter des lymphocytes T spécifiques du virus de l'hépatite C (VHC), a été testée *in vitro* par le dosage des cytokines IFN-γ, IL-2, IL-4 et IL-10 produites par les lymphocytes T issus de PBMC de patients infectés de façon chronique par le VHC.

De manière plus précise, les cellules mononucléées du sang périphérique (PBMC) de patients infectés de façon chronique par le VHC ont été séparées sur gradient de Ficoll, à partir d'un prélèvement sanguin hépariné. Les PBMC isolées ont ensuite été cultivées à 37°C, dans des plaques à 96 puits contenant du milieu RPMI 1640 (GIBCO) complet (10 % sérum humain 10 %, 100 UI/ml pénicilline et 10µg/ml streptomycine), seul ou en présence :
- d'un mélange des peptides C 19-47 et 31-57, d'un mélange des peptides C 127-149 et 148-173 ou des peptides NS3 1007-1037, 1036-1055, 1074-1195, 1190-1212, 1377-13403, 1524-1553, 1552-1583, chacun à la concentration de 10 µg/ml,
- d'un mélange de l'ensemble de ces peptides, chacun à la concentration de 110 µg/ml, 55 µg/ml ou 11 µg/ml, ou
- de phytohémagglutinine (PHA) ou de toxine tétanique (TT), chacune à la concentration de 2,5 µg/ml.

Les surnageants de culture ont été récoltés à 24h ou 48h et stockés à -80 °C jusqu'à l'analyse.

Les cytokines éventuellement présentes dans les surnageants ont été dosées de la façon suivante :

Des plaques à 96 puits (Maxisorp, NUNC) ont été recouvertes de 100 µl d'anticorps anti-IFN-γ, IL-2, IL-4 et IL-10 (BECTON DICKINSON, 2µg/ml) dans du tampon carbonate 0,1M, pH 8, 6 et incubées 2h à 37°C. Après trois lavages successifs avec du tampon PBS contenant 0,05 % de Twen 20, les plaques ont été saturées avec 200 µl de PBS contenant 3 % de sérum albumine bovine (BSA, SIGMA), pendant 2h à température ambiante. 50 µl de surnageant de culture (en double), ainsi qu'une une gamme étalon de cytokines recombinantes humaines (rH-IFN-γ, rH-IL-2, rH-IL-4 et rH-IL-10 ; BECTON DICKINSON) ont ensuite été ajoutés dans chaque puits et les plaques ont été incubées pendant 12h à + 4°C. Après trois lavages successifs avec du tampon PBS contenant 0,05 % de Twen 20, 100 µl d'anticorps secondaires anti-IFN-γ, IL-2, IL-4 et IL-10 (BECTON DICKINSON, 1µg/ml) dans du tampon PBS contenant 1 % de BSA ont été ajoutés dans les puits puis les plaques ont été incubées 1h à température ambiante. Après quatre lavages successifs avec du tampon PBS contenant 0,05 % de Twen 20, 100 µl de streptavidine-peroxydase (SIGMA) diluée au 1/10000 dans du tampon PBS contenant 0,1 % BSA ont été ajoutés dans les puits, puis les plaques ont été incubées 30 min à température ambiante. Après quatre lavages successifs avec du tampon PBS contenant 0,05 % de Twen 20, 100 µl d'orthophényldiamine (OPD, SIGMA, 400 µg/ml) dans du tampon citrate 50 mM ont été ajoutés dans les puits et les plaques ont été incubées à température ambiante, puis la réaction a été stoppée par ajout de 50 µl d'HCl 2N. La densité optique a ensuite été mesurée à 540 nM et l'analyse quantitative des cytokines a été réalisée à l'aide du logiciel Deltasoft (DS3-1.518F/1994 E. BERCHTOLD & BIOMETALLICS INC). Les résultats, exprimés en pg/ml, sont présentés au Tableau X.

**Tableau X: Dosage de cytokines produites par les lymphocytes T de patients infectés chroniques, après stimulation par les peptides C et NS3, seuls ou en mélange.**

| | | **-** | **PHA** | **TT** | **M110** | **M55** | **M11** | **1C + 2C** | **5C + 6C** | **8N** | **9N** | **15N** | **16N** | **23N** | **28N** | **29N** |
|---|---|---|---|---|---|---|---|---|---|---|---|---|---|---|---|---|
| patient 815 | IL2 | _ | 1002 | 610 | _ | _ | _ | _ | _ | _ | _ | _ | 72 | 58 | | |
| | IFNγ | _ | 1979 | _ | _ | _ | _ | _ | _ | _ | _ | _ | _ | _ | _ | _ |
| | IL4 | _ | 5638 | _ | _ | _ | _ | _ | _ | _ | _ | _ | _ | _ | _ | _ |
| | IL10 | _ | 497 | _ | 79 | 27 | _ | 11* | _ | _ | 156 | _ | 123 | 63* | _ | _ |
| patient 829 | IL2 | _ | NT | _6 | 3 | 3 | 3 | _ | _ | _ | _ | _ | _ | _ | _ | _ |
| | IFNγ | _ | NT | 441 | _ | _ | _ | _ | _ | _ | _ | _ | _ | _ | _ | _ |
| | IL4 | _ | NT | _ | _ | _ | _ | _ | _ | _ | _ | _ | _ | _ | _ | _ |
| | IL10 | NT | NT | _ | _ | _ | _ | _ | _ | _ | _ | _ | _ | _ | 67 | _ |
| patient 342 | IL2 | _ | 125 | 22 | 20 | _ | _ | _ | _ | _ | _ | _ | _ | _ | _ | _ |
| | IFNγ _ | _ | _ | _ | _ | _ | _ | _ | _ | _ | _ | _ | _ | _ | _ | _ |
| | IL4 | _ | _ | _ | _ | _ | _ | _ | _ | _ | _ | _ | _ | _ | _ | _ |
| | IL10 | NT | NT | NT | NT | NT | NT | NT | NT | NT | NT | NT | NT | NT | NT | NT |
| patient 862 | IL2 | _ | 60 | _ | _4 | 8* | _6 | _ | _ | _ | 29.1 | _ | _ | _ | _ | _ |
| | IFNγ | _ | 9902 | _ | _ | _ | _ | _ | _ | _ | _ | _ | _ | _ | _ | _ |
| | IL4 | _ | 9 | _ | _ | _ | _ | _ | _ | _ | _ | _ | _ | _ | _ | 57 |
| | IL10 | NT | NT | NT | NT | NT | NT | NT | NT | NT | NT | NT | NT | NT | NT | NT |
| patient 093 | IL2 | _ | 1402* | 171 | 7 | 8 | 7 | _ | 2 | _ | _ | _ | _ | _ | _ | _ |
| | IFNγ | _ | 8542 | 83 | _ | _ | _ | _ | 137 | _ | _ | _ | _ | 57 | _ | _ |
| | IL4 | _ | 127 | 24 | _ | _ | _ | 8 | 35 | _ | _ | _ | _ | 23 | 11 | 44 |
| | IL10 | _ | 8551 | _ | _ | _ | _ | _ | _ | _ | _ | _ | _ | _ | _ | _ |
| patient 067 | IL2 | _ | 1337 | 695 | 136 | 130 | 159 | _ | 100 | _ | _ | _ | _85 | _51 | _76 | 60 |
| | IFNγ | _ | 2447 | 212 | _ | _ | _ | _ | _ | _ | _ | _ | _ | _ | _ | _ |
| | IL4 | _ | 632 | _ | _ | _ | _ | _ | _ | _ | _ | _ | _ | _ | _ | _ |
| | IL10 | _ | 665 | _ | _ | _ | _ | _ | 32 | 43 | 58 | 55 | _ | _ | 86 | 31* |
| patient 630 | IL2 | _ | 18874 | 55 | 82 | 2,4 | 3,4 | _ | 12* | _ | _ | _ | _ | _ | _ | 2,4* |
| | IFNγ | _ | 474 | _ | _ | _ | _ | _ | _ | _ | _ | _ | _ | _ | _ | _ |
| | IL4 | _ | 251 | _ | _ | _ | _ | _ | _ | _ | _ | _ | _ | _ | _ | _ |
| | IL10 | _ | 242 | _ | _ | _ | _ | _ | _ | _ | _ | _ | _ | _ | _ | _ |
| patient 078 | IL2 | _ | 207 | 48 | _ | _ | 44 | _ | _ | _ | _ | _ | _17 | _ | _ | _ |
| | IFNγ | _ | 2249 | _ | _ | _ | _ | _ | _ | _ | _ | _ | _ | _ | _ | _ |
| | IL4 | _ | 55 | _ | _ | _ | _ | _ | _ | _ | _ | _ | _ | _ | _ | _ |
| | IL10 | _ | 207 | _ | 64 | _109 | 85 | 64 | 16 | 41 | 58 | 43 | | 54 | 21 | 38 |
| patient 659 | IL2 | _ | 153_ | _ | _ | _ | _ | _ | _ | _ | _ | _ | _ | _ | _ | _ |
| | IFNγ | _ | _187 | _ | _ | _ | _ | _ | _ | _ | _ | _ | _ | _ | _ | _ |
| | IL4 | _ | 453 | _ | _ | _ | _ | _ | _ | _ | _ | _ | _ | _ | _ | _ |
| | IL10 | _ | 182 | _ | 129 | 40 | | 31 | _ | _ | 18 | _ | _ | 29 | _ | _ |

| | | | | | | | | | | | | | | | | |
|---|---|---|---|---|---|---|---|---|---|---|---|---|---|---|---|---|
| * dosage à 24h au lieu de 48 h ; NT: non testé; - : non détecté | | | | | | | | | | | | | | | | |

Le Tableau X montre que 8 patients sur 9 présentent des cellules spécifiques d'au moins un des peptides C ou NS3, sécrétrices d'IL-2, IFN-γ, d'IL-4 ou d'IL-10. Il montre également qu'un mélange de peptides C et NS3 permet de détecter des lymphocytes T spécifiques du VHC au moins aussi efficacement que les peptides utilisés séparément, et qu'il augmente même la sensibilité de cette détection (voir patient 342).

Ces résultats indiquent que les peptides ayant une bonne affinité pour les molécules HLA II tels que définis ci-dessus, peuvent être utilisés dans un test de diagnostic de l'état immunitaire de patients vis-à-vis de l'infection par le VHC.

## Revendications

1. Mélange de peptides incluant au moins deux peptides différents issus du virus de l'hépatite C pour utilisation comme vaccin contre l'infection par le virus de l'hépatite C, **caractérisé en ce que** l'un au moins des peptides est un peptide issu de la protéine C qui se lie avec une activité de liaison < 1000 nM, à au moins quatre molécules HLA II différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4, lequel peptide étant sélectionné dans le groupe constitué par :
a) le peptide correspondant aux positions 127-167,
b) les peptides d'au moins 11 acides aminés comprenant les positions 134 à 145, inclus dans le peptide tel que défini en a), et
c) les peptides dérivés des peptides tels que définis en a) ou en b) par substitution par des résidus alanine (C → A), de résidu(s) cystéine situé(s) en position + 1 ou + 2, relativement au résidu d'acide aminé en position N-terminale et/ou en position - 1, - 2 ou - 3, relativement au résidu d'acide aminé en position C terminale.

2. Mélange de peptides selon la revendication 1, **caractérisé en ce que** les peptides d'au moins 11 acides aminés tels que définis en b) sont sélectionnés dans le groupe constitué par les peptides correspondant respectivement aux positions 127-149, 131-145, 13-1-148, 131-167 et 134-148.

3. Mélange de peptides selon la revendication 1, **caractérisé en ce que** le peptide tel que défini en c) est le peptide de séquence SEQ ID NO: 5, dérivé du peptide C 127-149.

4. Mélange de peptides selon l'une quelconque des revendications 1 à 3, **caractérisé en ce que** les dites molécules HLA II sont codées respectivement par les allèles HLA DRB1*0101, DRB1*0301, DRB1*0101, DRB1*0701, DRB1*1101, DRB1*1301, DRB1*1501, DRB3*0101, DRB4*0101, DRB5*0101, DP*0401 et DP*0402.

5. Mélange de peptides selon l'une quelconque des revendications 1 à 4, **caractérisé en ce qu'**il inclut, outre ledit peptide issu de la protéine C, au moins un peptide issu de la protéine NS3, se liant à au moins quatre molécules HLA II différentes dont la fréquence allélique est supérieure à 5 % dans la population caucasienne, avec une activité de liaison < 1000 nM.

6. Mélange de peptides selon la revendication 5, **caractérisé en ce que** les peptides issus de la protéine NS3, sont sélectionnés dans le groupe constitué par :
d) les peptides correspondant respectivement aux positions 1007-1037, 1036-1055, 1052-1072, 1076-1093, 1127-1153, 1149-1172, 1174-1195, 1190-1212, 1206-1239, 1246-1275, 1275-1304, 1361-1387, 1377-1403, 1404-1432, 1456-1481, 1495-1513, 1524-1553 et 1552-1583,
e) les peptides d'au moins 11 acides aminés inclus dans les peptides précédents, et
f) les peptides dérivés des peptides tels que définis en d) ou en e) par substitution par des résidus alanine (C → A), de résidu(s) cystéine situé(s) en position + 1 ou + 2, relativement au résidu d'acide aminé en position N-terminale et/ou en position - 1, - 2 ou - 3, relativement au résidu d'acide aminé en position C terminale..

7. Mélange de peptides selon la revendication 6, **caractérisé en ce que** les peptides d'au moins 11 acides aminés tels que définis en e) sont sélectionnés dans le groupe constitué par :
- les peptides inclus dans le peptide 1007-1037 correspondant respectivement aux positions 1007-1021, 1015-1029, 1015-1037, 1019-1033 et 1020-1034,
- les peptides inclus dans le peptide 1174-1195 correspondant respectivement aux positions 1174-1188, 1174-1192 et 1178-1192,
- les peptides inclus dans le peptide 1190-1212 correspondant respectivement aux positions 1190-1204 et 1192-1206,
- les peptides inclus dans le peptide 1246-1275 correspondant respectivement aux positions 1246-1260, 1246-1264, 1250-1264 et 1261-1275,
- les peptides inclus dans le peptide 1377-1403 correspondant respectivement aux positions 1381-1395, 1381-1397, 1381-1403 et 1383-1397,
- le peptide inclus dans le peptide 1495-1513 correspondant respectivement aux positions 1495-1509,
- les peptides inclus dans le peptide 1524-1553 correspondant respectivement aux positions 1524-1552, 1524-1538, 1528-1542, 1528-1552, 1529-1543, 1534-1548, 1538-1552 et 1540-1553, et
- les peptides inclus dans le peptide 1552-1583 correspondant respectivement aux positions 1559-1573 et 1563-1577.

8. Mélange de peptides selon la revendication 7, **caractérisé en ce que** les peptides tels que définis en f) sont sélectionnés dans le groupe constitué par les séquences SEQ ID NO: 10, 13, 20, 22 et 24 et les séquences dérivées de la séquence SEQ ID NO: 24 correspondant respectivement aux positions 1524-1538, 1524-1552, 1528-1552, 1538-1552 et 1540-1553.

9. Mélange de peptides selon l'une quelconque des revendications 1 à 8, **caractérisé en ce qu'**il inclut des peptides issus des protéines C et NS3 du Virus de l'hépatite C de génotype 1.

10. Mélange de peptides selon l'une quelconque des revendications 1 à 9, **caractérisé en ce qu'**il inclut 2 à 6 peptides différents issus des protéines C et NS3, l'ensemble des peptides se liant à au moins 10 molécules HLA II dont la fréquence allélique est supérieure à 5 % dans la population caucasienne.

11. Mélange de peptides selon la revendication 10, **caractérisé en ce qu'**il inclut des peptides sélectionnés dans le groupe constitué par les peptides issus de la protéine C correspondant respectivement aux positions 27-51, 131-167, 127-149, 131-148, 148-167 et les peptides issus de la protéine NS3 correspondant respectivement aux positions 1007-1037, 1015-1037, 1036-1055, 1174-1192, 1190-1212, 1246-1264, 1381-1403, 1381-1397, 1524-1553, 1528-1552 et 1552-1583.

12. Mélange de peptides selon l'une quelconque des revendications 1 à 11, **caractérisé en ce qu'**il est sous forme d'une protéine de fusion comprenant la séquence desdits peptides.

13. Molécule d'acide nucléique, **caractérisée en ce qu'**elle code pour une protéine de fusion selon la revendication 12.

14. Vecteur recombinant, **caractérisé en ce qu'**il comprend une molécule d'acide nucléique selon la revendication 13.

15. Cellule, **caractérisée en ce qu'**elle est transformée par un vecteur selon la revendication 14.

16. Composition immunogène anti-VHC, **caractérisée en ce qu'**elle comprend au moins :
- un mélange de peptides selon l'une quelconque des revendications 1 à 12, et/ou
- une molécule d'acide nucléique selon la revendication 13, ou
- un vecteur recombinant selon la revendication 14,
en association avec au moins un véhicule pharmaceutiquement acceptable et éventuellement au moins un adjuvant.

17. Composition immunogène selon la revendication 16, **caractérisée en ce que** lesdits peptides sont sous forme de peptides modifiés ou bien associés à des liposomes ou à des lipides, notamment sous forme de lipopeptides.

18. Composition immunogène selon la revendication 16 ou la revendication 17, **caractérisée en ce que** ledit mélange de peptides est associé :
- à un ou plusieurs peptides ou lipopeptides contenant un ou plusieurs épitopes CD8+ et plus particulièrement des épitopes CD8+ issus d'une protéine du VHC tels que les peptides C2-10, 28-36, 35-44, 41-49, 42-50, 85-98, 88-97, 127-140, 131-140, 132-140, 167-176, 178-187, 181-190 ; les peptides E1 220-227, 233-242, 234-242, 363-371 ; les peptides E2 441-411, 460-469, 489-496, 569-578, 621-628, 725-733 ; les peptides NS2 826-838, 838-845 ; les peptides NS3 1073-1081, 1169-1177, 1287-1296, 1395-1403, 1406-1415 ; les peptides NS4A 1585-1593, 1666-1675 ; les peptides NS4B 1769-1777, 1789-1797, 1807-1816, 1851-1859; le peptide NS5A 2252-2260 et les peptides NS5B 2588-2596, 2727-2735.
- à d'autres peptides comprenant des épitopes CD4+ multiples, tels que le peptide de la toxine tétanique TT (positions 830-846), le peptide de l'hémagglutinine *d'Influenza* HA (positions 307-319), PADRE et le peptide LSA3 de *Plasmodium falciparum* et/ou
- à un ou plusieurs peptides ou lipopeptides contenant un ou plusieurs épitopes B, plus particulièrement des épitopes B issus d'une protéine du VHC reconnus spécifiquement par des anticorps dirigés contre ces derniers, tels que le peptide C5-27, le peptide NS4 1698-1719 et le peptide NS5 2295-2315.

19. Vaccin, **caractérisé en ce qu'**il inclut une composition immunogène selon l'une quelconque des revendications 16 à 18.

20. Mélange de peptides tel que défini à l'une quelconque des revendications 1 à 12.

21. Peptide tel que défini à l'une quelconque des revendications 1, 2, 3 et 9, à l'exclusion des peptides C131-150 et C133-152.

22. Réactif de diagnostic, **caractérisé en ce qu'**il comprend au moins un peptide selon la revendication 21 ou un mélange de peptides selon la revendication 20, éventuellement marqué ou complexé, sous la forme de complexes multimériques.

23. Procédé *in vitro* d'évaluation de l'état immunitaire d'un individu, **caractérisé en ce qu'**il comprend une étape de détection de la présence de cellules T CD4+ spécifiques du VHC, à l'aide d'un réactif de diagnostic selon la revendication 22.

24. Méthode de tri de lymphocytes T spécifiques du VHC, **caractérisé en ce qu'**il comprend au moins les étapes suivantes :
- mise en contact, d'une suspension de cellules à trier avec un ou plusieurs tétramères marqués formés à partir de complexes entre les peptides ou les mélanges de peptides tels que définis à la revendication 22, et des molécule HLA II solubles , et
- tri des cellules marquées par les tétramères.

## Claims

1. Mixture of peptides including at least two different peptides obtained from the hepatitis C virus for use as a vaccine against infection by the hepatitis C virus, **characterised in that** at least one of the peptides is a peptide obtained from the protein C which binds, with a binding activity < 1000 nM, to at least four different HLA II molecules selected from among the HLA-DR1, HLA-DR3, HLA-DR4, HLA-DR7, HLA-DR11, HLA-DR13, NLA-DR15, HLA-DRB3, HLA-DRB4, HLA-DRB5 and HLA-DP4 molecules, said peptide being selected from the group consisting of:
a) the peptide corresponding to positions 127-167,
b) the peptides of at least 11 amino acids comprising positions 134 to 145, included in the peptide as defined in a), and
c) the peptides derived from the peptides as defined in a) or b) by substitution by alanine residues (C →A), of cysteine residue(s) situated in the +1 or +2 position, relative to the amino acid residue in the N-terminal position, and/or in the -1, -2 or -3 position, relative to the amino acid residue in the C-terminal position.

2. Mixture of peptides according to claim 1, **characterised in that** the peptides of at least 11 amino acids as defined in b) are selected from the group consisting of the peptides corresponding to positions 127-149, 131-145, 131-148, 131-167 and 134-148, respectively.

3. Mixture of peptides according to claim 1, **characterised in that** the peptide as defined in c) is the peptide of sequence SEQ ID NO: 5, derived from the peptide C 127-149.

4. Mixture of peptides according to any one of claims 1 to 3, **characterised in that** said HLA II molecules are coded by the alleles HLA DRB1*0101, DRB1*0301, DRB1*0101, DRB1*0701, DRB1*1101, DRB1*1301, DRB1*1501, DRB3*0101, DRB4*0101, DRB5*0101, DP*0401 and DP*0402, respectively.

5. Mixture of peptides according to any one of claims 1 to 4, **characterised in that** it includes, besides the peptide obtained from protein C, at least one peptide obtained from protein NS3, binding to at least four different HLA II molecules the allelic frequency of which is greater than 5% in the Caucasian population, with a binding activity < 1000 nM.

6. Mixture of peptides according to claim 5, **characterised in that** the peptides obtained from protein NS3 are selected from the group consisting of:
d) the peptides corresponding to positions 1007-1037, 1036-1055, 1052-1072, 1076-1093, 1127-1153, 1149-1172, 1174-1195, 1190-1212, 1206-1239, 1246-1275, 1275-1304, 1361-1387, 1377-1403, 1404-1432, 1456-1481, 1495-1513, 1524-1553 and 1552-1583, respectively,
e) the peptides of at least 11 amino acids included in the above-mentioned peptides, and
f) the peptides derived from the peptides as defined in d) or e) by substitution by alanine residues (C →A), of cysteine residue(s) situated in the +1 or +2 position, relative to the amino acid residue in the N-tenninal position, and/or in the -1, -2 or -3 position, relative to the amino acid residue in the C-terminal position.

7. Mixture of peptides according to claim 6, **characterised in that** the peptides of at least 11 amino acids as defined in e) are selected from the group consisting of:
- the peptides included in the peptide 1007-1037 corresponding to positions 1007-1021, 1015-1029, 1015-1037, 1019-1033 and 1020-1034, respectively,
- the peptides included in the peptide 1174-1195, corresponding to positions 1174-1188, 1174-1192 and 1178-1192, respectively,
- the peptides included in the peptide 1190-1212, corresponding to positions 1190-1204 and 1192-1206, respectively,
- the peptides included in the peptide 1246-1275, corresponding to positions 1246-1260, 1246-1264, 1250-1264 and 1261-1275, respectively,
- the peptides included in the peptide 1377-1403, corresponding to positions 1381-1395, 1381-1397, 1381-1403 and 1383-1397, respectively,
- the peptide included in the peptide 1495-1513 corresponding to positions 1495-1509, respectively,
- the peptides included in the peptide 1524-1553 corresponding to positions 1524-1552, 1524-1538, 1528-1542, 1528-1552, 1529-1543, 1534-1548, 1538-1552 and 1540-1553, respectively, and
- the peptides included in the peptide 1552-1583 corresponding to positions 1559-1573 and 1563-1577, respectively.

8. Mixture of peptides according to claim 7, **characterised in that** the peptides as defined in f) are selected from the group comprising the sequences SEQ ID NO: 10, 13, 20, 22 and 24, and the sequences derived from the sequence SEQ ID NO: 24 corresponding to positions 1524-1538, 1524-1552, 1528-1552, 1538-1552 and 1540-1553, respectively.

9. Mixture of peptides according to any one of claims 1 to 8, **characterised in that** it includes peptides obtained from the proteins C and NS3 of the hepatitis C virus of genotype 1.

10. Mixture of peptides according to any one of claims 1 to 9, **characterised in that** it includes 2 to 6 different peptides obtained from proteins C and NS3, all the peptides binding to at least 10 HLA II molecules the allelic frequency of which is greater than 5% in the Caucasian population.

11. Mixture of peptides according to claim 10, **characterised in that** it includes peptides selected from the group consisting of the peptides obtained from protein C corresponding to positions 27-51, 131-167, 127-149, 131-148, 148-167, respectively, and the peptides obtained from the protein NS3 corresponding to positions 1007-1037, 1015-1037, 1036-1055, 1174-1192, 1190-1212, 1246-1264, 1381-1403, 1381-1397, 1524-1553, 1528-1552 and 1552-1583, respectively.

12. Mixture of peptides according to any one of claims 1 to 11, **characterised in that** it is in the form of a fusion protein comprising the sequence of said peptides.

13. Nucleic acid molecule, **characterised in that** it codes for a fusion protein according to claim 12.

14. Recombinant vector, **characterised in that** it comprises a nucleic acid molecule according to claim 13.

15. Cell, **characterised in that** it is transformed by a vector according to claim 14.

16. Immunogenic anti-HCV composition, **characterised in that** it comprises at least:
- a mixture of peptides according to any one of claims 1 to 12, and/or
- a nucleic acid molecule according to claim 13, or
- a recombinant vector according to claim 14,
combined with at least one pharmaceutically acceptable carrier and optionally at least one adjuvant.

17. Immunogenic composition according to claim 16, **characterised in that** the peptides are in the form of modified peptides or are associated with liposomes or lipids, notably in the form of lipopeptides.

18. Immunogenic composition according to claim 16 or 17, **characterised in that** the mixture of peptides is associated with:
- one or more peptides or lipopeptides containing one or more CD8+ epitopes and more particularly CD8+ epitopes obtained from a protein of HCV such as the peptides C2-10, 28-36, 35-44, 41-49, 42-50, 85-98, 88-97, 127-140, 131-140, 132-140, 167-176, 178-187, 181-190; the E1 peptides 220-227, 233-242, 234-242, 363-371; the E2 peptides 401-411, 460-469, 489-496, 569-578, 621-628, 725-733; the NS2 peptides 826-838, 838-845; the NS3 peptides 1073-1081, 1169-1177, 1287-1296, 1395-1403, 1406-1415; the NS4A peptides 1585-1593, 1666-1675; the NS4B peptides 1769-1777, 1789-1797, 1807-1816, 1851-1859; the NS5A peptide 2252-2260 and the NS5B peptides 2588-2596, 2727-2735,
- other peptides comprising multiple CD4+ epitopes, such as the tetanus toxin TT peptide (positions 830-846), the *Influenza* haemagglutinin HA peptide (positions 307-319), PADRE and the *Plasmodium falciparum* LSA3 peptide, and/or
- one or more peptides or lipopeptides containing one or more B epitopes, more particularly B epitopes obtained from an HCV protein which are specifically recognised by antibodies directed against them, such as peptide C5-27, peptide NS4 1698-1719 and peptide NS5 2295-2315.

19. Vaccine, **characterised in that** it includes an immunogenic composition according to any one of claims 16 to 18,

20. Mixture of peptides as defined in any one of claims 1 to 12.

21. Peptide as defined in any one of claims 1, 2, 3 and 9, with the exception of peptides C131-150 and C133-152.

22. Diagnostic reagent, **characterised in that** it comprises at least one peptide according to claim 21, or a mixture of peptides according to claim 20, optionally labelled or complexed, in the form of multimeric complexes.

23. Process for evaluating *in vitro* the immune state of an individual, **characterised in that** it comprises a step of detecting the presence of CD4+ T cells specific to HCV, with the aid of a diagnostic reagent according to claim 22.

24. Method of sorting T lymphocytes specific to HCV, **characterised in that** it comprises at least the following steps:
- contacting a cell suspension which is to be sorted with one or more labelled tetramers formed from complexes between the peptides or mixtures of peptides as defined in claim 22, and soluble HLA II molecules, and
- sorting the cells labelled by the tetramers.

## Patentansprüche

1. Mischung von Peptiden, die wenigstens zwei unterschiedliche Peptide enthält, die von Hepatitis C-Virus stammen, zur Verwendung als Impfstoff gegen Hepatitis C-Virus-Infektion, **dadurch gekennzeichnet, dass** wenigstens eines der Peptide ein aus Protein C stammendes Peptid ist, das mit einer Bindungsaktivität von < 1000 nM an wenigstens vier unterschiedliche HLA II-Moleküle bindet, die ausgewählt sind aus den Molekülen HLA-DR1, HLA-DR3, HLA-DR4, HLA-DR7, HLA-DR11, HLA-DR13, HLA-DR15, HLA-DRB3, HLA-DRB4, HLA-DRB5 und HLA-DP4, wobei das Peptid ausgewählt ist aus der Gruppe bestehend aus:
a) dem Peptid, das den Positionen 127-167 entspricht,
b) den Peptiden, die in dem Peptid gemäß a) enthalten sind, mit wenigstens 11 Aminosäuren, die die Positionen 134-145 umfassen, und
c) den Peptiden, die sich von den Peptiden gemäß a) oder b) durch Substitution von ein oder mehr Cysteinresten, die sich relativ zum Aminosäurerest in N-terminaler Position an Position +1 oder +2 und/oder relativ zum Aminosäurerest in C-terminaler Position an Position -1, -2 oder -3 befinden, durch Alaninreste (C → A) ableiten.

2. Mischung von Peptiden nach Anspruch 1, **dadurch gekennzeichnet, dass** die Peptide mit wenigstens 11 Aminosäuren gemäß b) ausgewählt sind aus der Gruppe bestehend aus den Peptiden, die den Positionen 127-149, 131-145, 131-148, 131-167 bzw. 134-148 entsprechen.

3. Mischung von Peptiden nach Anspruch 1, **dadurch gekennzeichnet, dass** das Peptid gemäß c) das Peptid der Sequenz SEQ ID NO:5 ist, das von Peptid C 127-149 abgeleitet ist.

4. Mischung von Peptiden nach irgendeinem der Ansprüche 1 bis 3, **dadurch gekennzeichnet, dass** die HLA 11-Moleküle von den Allelen HLA DRB1*0101, DRB1*0301, DRB1*0401, DRB1*0701, DRB1*1101, DRB1*1301, DRB1*1501, DRB3*0101, DRB4*0101, DRB5*0101, DP*0401 bzw. DP*0402 codiert werden.

5. Mischung von Peptiden nach irgendeinem der Ansprüche 1 bis 4, **dadurch gekennzeichnet, dass** sie neben dem Peptid, das aus Protein C stammt, wenigstens ein aus dem NS3 Protein stammendes Peptid beinhaltet, das mit einer Bindungsaktivität von < 1000 nM an wenigstens vier unterschiedliche HLA 11-Moleküle bindet, deren Allelfrequenz in der kaukasischen Population über 5 % beträgt.

6. Mischung von Peptiden nach Anspruch 5, **dadurch gekennzeichnet, dass** die aus dem NS3 Protein stammenden Peptide ausgewählt sind aus der Gruppe bestehend aus:
d) den Peptiden, die den Positionen 1007-1037, 1036-1055, 1052-1072, 1076-1093, 1127-1153, 1149-1172, 1174-1195, 1190-1212, 1206-1239, 1246-1275, 1275-1304, 1361-1387, 1377-1403, 1404-1432, 1456-1481, 1495-1513, 1524-1553 bzw. 1552-1583 entsprechen,
e) den in den vorhergehenden Peptiden enthaltenen Peptiden mit wenigstens 11 Aminosäuren, und
f) den Peptiden, die sich von den Peptiden gemäß d) oder e) durch Substitution von ein oder mehr Cysteinresten, die sich relativ zum Aminosäurerest in N-terminaler Position an Position +1 oder +2 und/oder relativ zum Aminosäurerest in C-terminaler Position an Position -1, -2 oder -3 befinden, durch Alaninreste (C → A) ableiten.

7. Mischung von Peptiden nach Anspruch 6, **dadurch gekennzeichnet, dass** die Peptide mit wenigstens 11 Aminiosäuren gemäß e) ausgewählt sind aus der Gruppe bestehend aus:
- den in Peptid 1007-1037 enthaltenen Peptiden, die den Positionen 1007-1021, 1015-1029, 1015-1037, 1019-1033 bzw. 1020-1034 entsprechen,
- den in Peptid 1174-1195 enthaltenen Peptiden, die den Positionen 1174-1188, 1174-1192 bzw. 1178-1192 entsprechen,
- den in Peptid 1190-1212 enthaltenen Peptiden, die den Positionen 1190-1204 bzw. 1192-1206 entsprechen,
- den in Peptid 1246-1275 enthaltenen Peptiden, die den Positionen 1246-1260, 1246-1264, 1250-1264 bzw. 1261-1275 entsprechen,
- den in Peptid 1377-1403 enthaltenen Peptiden, die den Positionen 1381-1395, 1381-1397, 1381-1403 bzw. 1383-1397 entsprechen,
- dem in Peptid 1495-1513 enthaltenen Peptid, das den Positionen 1495-1509 entspricht,
- den in Peptid 1524-1553 enthaltenen Peptiden, die den Positionen 1524-1552, 1524-1538, 1528-1542, 1528-1552, 1529-1543, 1534-1548, 1538-1552 bzw. 1540-1553 entsprechen, und
- den in Peptid 1552-1583 enthaltenen Peptiden, die den Positionen 1559-1573 bzw. 1563-1577 entsprechen.

8. Mischung von Peptiden nach Anspruch 7, **dadurch gekennzeichnet, dass** die Peptide gemäß f) ausgewählt sind aus der Gruppe bestehend aus den Sequenzen SEQ ID NO:10, 13, 20, 22 und 24 und den von der Sequenz SEQ ID NO:24 abgeleiteten Sequenzen, die den Positionen 1524-1538, 1524-1552, 1528-1552, 1538-1552 bzw. 1540-1553 entsprechen.

9. Mischung von Peptiden nach irgendeinem der Ansprüche 1 bis 8, **dadurch gekennzeichnet, dass** sie Peptide enthält, die aus den Proteinen C und NS3 des Hepatitis C-Virus des Genotyps 1 stammen.

10. Mischung von Peptiden nach irgendeinem der Ansprüche 1 bis 9, **dadurch gekennzeichnet, dass** sie 2 bis 6 unterschiedliche Peptide enthält, die aus den Proteinen C und NS3 stammen, wobei die Peptide insgesamt an wenigstens 10 HLA II-Moleküle binden, deren Allelfrequenz in der kaukasischen Population über 5 % liegt.

11. Mischung von Peptiden nach Anspruch 10, **dadurch gekennzeichnet, dass** sie Peptide enthält, die ausgewählt sind aus der Gruppe bestehend aus den aus Protein C stammenden Peptiden, die den Positionen 27-51, 131-167, 127-149, 131-148 bzw. 148-167 entsprechen, und den aus dem NS3-Protein stammenden Peptiden, die den Positionen 1007-1037, 1015-1037, 1036-1055, 1174-1192, 1190-1212, 1246-1264, 1381-1403, 1381-1397, 1524-1553, 1528-1552 bzw. 1552-1583 entsprechen.

12. Mischung von Peptiden nach irgendeinem der Ansprüche 1 bis 11, **dadurch gekennzeichnet, dass** sie in Form eines Fusionsproteins vorliegt, das die Sequenzen dieser Peptide umfasst.

13. Nukleinsäuremolekül, **dadurch gekennzeichnet, dass** es für ein Fusionsprotein nach Anspruch 12 codiert.

14. Rekombinanter Vektor, **dadurch gekennzeichnet, dass** er ein Nukleinsäuremolekül nach Anspruch 13 umfasst.

15. Zelle, **dadurch gekennzeichnet, dass** sie mit einem Vektor nach Anspruch 14 transformiert ist.

16. Anti-HCV-immunogene Zusammensetzung, **dadurch gekennzeichnet, dass** sie wenigstens umfasst:
- eine Mischung von Peptiden nach irgendeinem der Ansprüche 1 bis 12, und/oder
- ein Nukleinsäuremolekül nach Anspruch 13, oder
- einen rekombinanten Vektor nach Anspruch 14,
zusammen mit wenigstens einem pharmazeutisch verträglichen Träger und gegebenenfalls wenigstens einem Adjuvans.

17. Immunogene Zusammensetzung nach Anspruch 16, **dadurch gekennzeichnet, dass** die Peptide in Form von modifizierten Peptiden oder assoziiert mit Liposomen oder Lipiden vorliegen, insbesondere in Form von Lipopeptiden.

18. Immunogene Zusammensetzung nach Anspruch 16 oder Anspruch 17, **dadurch gekennzeichnet, dass** die Mischung von Peptiden assoziiert ist mit:
- ein oder mehreren Peptiden oder Lipopeptiden, die ein oder mehrere CD8+-Epitope und insbesondere CD8+-Epitope enthalten, die von einem HCV-Protein stammen, wie die Peptide C2-10, 28-36, 35-44, 41-49, 42-50, 85-98, 88-97, 127-140, 131-140, 132-140, 167-176, 178-187, 181-190; die Peptide E1 220-227, 233-242, 234-242, 363-371; die Peptide E2 401-411, 460-469, 489-496, 569-578, 621-628, 725-733; die Peptide NS2 826-838, 838-845; die Peptide NS3 1073-1081, 1169-1177, 1287-1296, 1395-1403, 1406-1415; die Peptide NS4A 1585-1593, 1666-1675; die Peptide NS4B 1769-1777, 1789-1797, 1807-1816, 1851-1859; das Peptid NS5A 2252-2260 und die Peptide NS5B 2588-2596, 2727-2735,
- weiteren Peptiden, die mehrere CD4+-Epitope umfassen, wie das Tetanustoxinpeptid TT (Positionen 830-846), das Hämagglutininpeptid von *Influenza* HA (Positionen 307-319), PADRE und das Peptid LSA3 von *Plasmodium falciparum,* und/oder
- ein oder mehreren Peptiden oder Lipopeptiden, die ein oder mehrere B-Epitope enthalten, insbesondere B-Epitope, die aus einem HCV-Protein stammen und spezifisch von Antikörpern erkannt werden, die gegen Letztere gerichtet sind, wie das Peptid C5-27, das Peptid NS4 1698-1719 und das Peptid NS5 2295-2315.

19. Impfstoff, **dadurch gekennzeichnet, dass** er eine immunogene Zusammensetzung nach irgendeinem der Ansprüche 16 bis 18 enthält.

20. Mischung von Peptiden gemäß irgendeinem der Ansprüche 1 bis 12.

21. Peptid gemäß irgendeinem der Ansprüche 1, 2, 3 und 9, ausgenommen die Peptide C131-140 und C133-152.

22. Diagnostisches Reagens, **dadurch gekennzeichnet, dass** es wenigstens ein Peptid nach Anspruch 21 oder eine Mischung von Peptiden nach Anspruch 20 umfasst, gegebenenfalls markiert oder komplexiert in Form multimerer Komplexe.

23. *In vitro*-Verfahren zur Bestimmung des Immunstatus eines Individuums, **dadurch gekennzeichnet, dass** es einen Schritt zum Nachweis der Anwesenheit von HCV-spezifischen CD4+-T-Zellen mit Hilfe eines diagnostischen Reagens nach Anspruch 22 umfasst.

24. Sortierverfahren für HCV-spezifische T-Lymphozyten, **dadurch gekennzeichnet, dass** es wenigstens die folgenden Schritte umfasst:
- Inkontaktbringen einer zu sortierenden Suspension von Zellen mit ein oder mehreren markierten Tetrameren, die aus Komplexen zwischen den Peptiden oder den Mischungen von Peptiden gemäß Anspruch 22 und löslichen HLA 11-Molekülen gebildet sind, und
- Aussortieren der mit den Tetrameren markierten Zellen.


### SEQUENCE LISTING

<110> COMMISSARIAT A L'ENERGIE ATOMIQUE
   SEDAC THERAPEUTICS
   MAILLERE Bernard
   GEORGES Bertrand
   CASTELLI FLorence
   BOUZIDI Ahmed
<120> MELANGE DE PEPTIDES ISSUS DES PROTEINES C ET NS3 DU VIRUS DE L'HEPATITE C ET LEURS APPLICATIONS
<130> BLO/HLP/cpF263/82FR
<160> 86
<170> PatentIn version 3.1
<210> 1
   <211> 29
   <212> PRT
   <213> Hepatitis C virus
<400> 1
   Pro Gln Asp Val Lys Phe Pro Gly Gly Gly Gln Ile Val Gly Gly Val
   1               5                   10                  15
   Tyr Leu Leu Pro Arg Arg Gly Pro Arg Leu Gly Val Arg
               20                  25
<210> 2
   <211> 27
   <212> PRT
   <213> Hepatitis C virus
<400> 2
   Val Gly Gly Val Tyr Leu Leu Pro Arg Arg Gly Pro Arg Leu Gly Val
   1               5                   10                  15
   Arg Ala Thr Arg Lys Thr Ser Glu Arg Ser Gln
               20                  25
<210> 3
   <211> 15
   <212> PRT
   <213> Hepatitis C virus
<400> 3
   Trp Ala Gly Trp Leu Leu Ser Pro Arg Gly Ser Arg Pro Ser Trp
   1               5                   10                  15
<210> 4
   <211> 30
   <212> PRT
   <213> Hepatitis C virus
<400> 4
   Arg Pro Ser Trp Gly Pro Thr Asp Pro Arg Arg Arg Ser Arg Asn Leu
   1               5                   10                  15
   Gly Lys Val Ile Asp Thr Leu Thr Cys Gly Phe Ala Asp Leu
               20                  25                  30
<210> 5
   <211> 23
   <212> PRT
   <213> Hepatitis C virus
<400> 5
   Thr Ala Gly Phe Ala Asp Leu Met Gly Tyr Ile Pro Leu Val Gly Ala
   1               5                   10                  15
   Pro Leu Gly Gly Ala Ala Arg
               20
<210> 6
   <211> 26
   <212> PRT
   <213> Hepatitis C virus
<400> 6
   Ala Arg Ala Leu Ala His Gly Val Arg Val Leu Glu Asp Gly Val Asn
   1               5                   10                  15
   Tyr Ala Thr Gly Asn Leu Pro Gly Ala Ser
               20                  25
<210> 7
   <211> 31
   <212> PRT
   <213> Hepatitis C virus
<400> 7
   Gly Arg Glu Ile Leu Leu Gly Pro Ala Asp Gly Met Val Ser Lys Gly
   1 5 10 15
   Trp Arg Leu Leu Ala Pro Ile Thr Ala Tyr Ala Gln Gln Thr Arg
   20 25 30
<210> 8
   <211> 20
   <212> PRT
   <213> Hepatitis C virus
<400> 8
   Thr Arg Gly Leu Leu Gly Cys Ile Ile Thr Ser Leu Thr Gly Arg Asp
   1 5 10 15
   Lys Asn Gln Val
   20
<210> 9
   <211> 21
   <212> PRT
   <213> Hepatitis C virus
<400> 9
   Lys Asn Gln Val Glu Gly Glu Val Gln Ile Val Ser Thr Ala Ala Gln
   1 5 10 15
   Thr Phe Leu Ala Thr
   20
<210> 10
   <211> 18
   <212> PRT
   <213> Hepatitis C virus
<400> 10
   Gly Val Ala Trp Thr Val Tyr His Gly Ala Gly Thr Arg Thr Ile Ala
   1 5 10 15
   Ser Pro
<210> 11
   <211> 26
   <212> PRT
   <213> Hepatitis C virus
<400> 11
   Lys Gly Pro Val Ile Gln Met Tyr Thr Asn Val Asp Gln Asp Leu Val
   1               5                   10                  15
   Gly Trp Pro Ala Pro Gln Gly Ser Arg Ser
               20                  25
<210> 12
   <211> 27
   <212> PRT
   <213> Hepatitis C virus
<400> 12
   Ser Ser Asp Leu Tyr Leu Val Thr Arg His Ala Asp Val Ile Pro Val
   1               5                   10                  15
   Arg Arg Arg Gly Asp Ser Arg Gly Ser Leu Leu
               20                  25
<210> 13
   <211> 24
   <212> PRT
   <213> Hepatitis C virus
<400> 13
   Arg Gly Ser Leu Leu Ser Pro Arg Pro Ile Ser Tyr Leu Lys Gly Ser
   1               5                   10                  15
   Ser Gly Gly Pro Leu Leu Ala Pro
               20
<210> 14
   <211> 22
   <212> PRT
   <213> Hepatitis C virus
<400> 14
   Gly His Ala Val Gly Ile Phe Arg Ala Ala Val Cys Thr Arg Gly Val
   1               5                   10                  15
   Ala Lys Ala Val Asp Phe
               20
<210> 15
   <211> 23
   <212> PRT
   <213> Hepatitis C virus
<400> 15
   Ala Lys Ala Val Asp Phe Ile Pro Val Glu Asn Leu Glu Thr Thr Met
   1 5 10 15
   Arg Ser Pro Val Phe Thr Asp
   20
<210> 16
   <211> 34
   <212> PRT
   <213> Hepatitis C virus
<400> 16
   Arg Ser Pro Val Phe Thr Asp Asn Ser Ser Pro Pro Val Val Pro Gln
                   5                   10                  15
   Ser Phe Gln Val Ala His Leu His Ala Pro Thr Gly Ser Gly Lys Ser
               20                  25                  30
   Thr Lys
<210> 17
   <211> 30
   <212> PRT
   <213> Hepatitis C virus
<400> 17
   Ala Gln Gly Tyr Lys Val Leu Val Leu Asn Pro Ser Val Ala Ala Thr
   1               5                   10                  15
   Leu Gly Phe Gly Ala Tyr Met Ser Lys Ala His Gly Ile Asp
               20                  25                  30
<210> 18
   <211> 30
   <212> PRT
   <213> Hepatitis C virus
<400> 18
   Asp Pro Asn Ile Arg Thr Gly Val Arg Thr Ile Thr Thr Gly Ser Pro
   1               5                   10                  15
   Ile Thr Tyr Ser Thr Tyr Gly Lys Phe Leu Ala Asp Gly Gly
               20                  25                  30
<210> 19
   <211> 26
   <212> PRT
   <213> Hepatitis C virus
<400> 19
   Ile Glu Glu Val Ala Leu Ser Thr Thr Gly Glu Ile Pro Phe Tyr Gly
   1               5                   10                  15
   Lys Ala Ile Pro Leu Glu Val Ile Lys Gly
               20                  25
<210> 20
   <211> 27
   <212> PRT
   <213> Hepatitis C virus
<400> 20
   Gly Lys Ala Ile Pro Leu Glu Val Ile Lys Gly Gly Arg His Leu Ile
   1               5                   10                  15
   Phe Cys His Ser Lys Lys Lys Ala Asp Glu Leu
               20                  25
<210> 21
   <211> 29
   <212> PRT
   <213> Hepatitis C virus
<400> 21
   Ala Ala Lys Leu Val Ala Leu Gly Ile Asn Ala Val Ala Tyr Tyr Arg
   1               5                   10                  15
   Gly Leu Asp Val Ser Val Ile Pro Thr Ser Gly Asp Val
               20                  25
<210> 22
   <211> 26
   <212> PRT
   <213> Hepatitis C virus
<400> 22
   Thr Ala Val Thr Gln Thr Val Asp Phe Ser Leu Asp Pro Thr Phe Thr
   1               5                   10                  15
   Ile Glu Thr Ile Thr Leu Pro Gln Asp Ala
               20                  25
<210> 23
   <211> 19
   <212> PRT
   <213> Hepatitis C virus
<400> 23
   Lys Pro Gly Ile Tyr Arg Phe Val Ala Pro Gly Glu Arg Pro Ser Gly
   1               5                   10                  15
   Met Phe Asp
<210> 24
   <211> 30
   <212> PRT
   <213> Hepatitis C virus
<400> 24
   Gly Ala Ala Trp Tyr Glu Leu Thr Pro Ala Glu Thr Thr Val Arg Leu
   1               5                   10                  15
   Arg Ala Tyr Met Asn Thr Pro Gly Leu Pro Val Ala Gln Asp
               20                  25                  30
<210> 25
   <211> 32
   <212> PRT
   <213> Hepatitis C virus
<400> 25
   Gln Asp His Leu Glu Phe Trp Glu Gly Val Phe Thr Gly Leu Thr His
   1               5                   10                  15
   Ile Asp Ala His Phe Leu Ser Gln Thr Lys Gln Ser Gly Glu Asn Leu
               20                  25                  30
<210> 26
   <211> 15
   <212> PRT
   <213> Hepatitis C virus
<400> 26
   Gly Arg Glu Ile Leu Leu Gly Pro Ala Asp Gly Met Val Ser Lys
   1 5 10 15
<210> 27
   <211> 15
   <212> PRT
   <213> Hepatitis C virus
<400> 27
   Leu Leu Gly Pro Ala Asp Gly Met Val Ser Lys Gly Trp Arg Leu
   1 5 10 15
<210> 28
   <211> 15
   <212> PRT
   <213> Hepatitis C virus
<400> 28
   Ala Asp Gly Met Val Ser Lys Gly Trp Arg Leu Leu Ala Pro Ile
   1 5 10 15
<210> 29
   <211> 15
   <212> PRT
   <213> Hepatitis C virus
<400> 29
   Val Ser Lys Gly Trp Arg Leu Leu Ala Pro Ile Thr Ala Tyr Ala
   1 5 10 15
<210> 30
   <211> 15
   <212> PRT
   <213> Hepatitis C virus
<400> 30
   Ser Lys Gly Trp Arg Leu Leu Ala Pro Ile Thr Ala Tyr Ala Gln
   1 5 10 15
<210> 31
   <211> 14
   <212> PRT
   <213> Hepatitis C virus
<400> 31
   Arg Leu Leu Ala Pro Ile Thr Ala Tyr Ala Gln Gln Thr Arg
   1 5 10
<210> 32
   <211> 15
   <212> PRT
   <213> Hepatitis C virus
<400> 32
   Gly His Ala Val Gly Ile Phe Arg Ala Ala Val Cys Thr Arg Gly
   1 5 10 15
<210> 33
   <211> 15
   <212> PRT
   <213> Hepatitis C virus
<400> 33
   Gly Ile Phe Arg Ala Ala Val Cys Thr Arg Gly Val Ala Lys Ala
   1 5 10 15
<210> 34
   <211> 15
   <212> PRT
   <213> Hepatitis C virus
<400> 34
   Arg Ala Ala Val Cys Thr Arg Gly Val Ala Lys Ala Val Asp Phe
   1 5 10 15
<210> 35
   <211> 15
   <212> PRT
   <213> Hepatitis C virus
<400> 35
   Gly Ala Ala Trp Tyr Glu Leu Thr Pro Ala Glu Thr Thr Val Arg
   1 5 10 15
<210> 36
   <211> 15
   <212> PRT
   <213> Hepatitis C virus
<400> 36
   Tyr Glu Leu Thr Pro Ala Glu Thr Thr Val Arg Leu Arg Ala Tyr
   1 5 10 15
<210> 37
   <211> 15
   <212> PRT
   <213> Hepatitis C virus
<400> 37
   Glu Leu Thr Pro Ala Glu Thr Thr Val Arg Leu Arg Ala Tyr Met
   1 5 10 15
<210> 38
   <211> 15
   <212> PRT
   <213> Hepatitis C virus
<400> 38
   Glu Thr Thr Val Arg Leu Arg Ala Tyr Met Asn Thr Pro Gly Leu
   1 5 10 15
<210> 39
   <211> 15
   <212> PRT
   <213> Hepatitis C virus
<400> 39
   Arg Leu Arg Ala Tyr Met Asn Thr Pro Gly Leu Pro Val Ala Gln
   1 5 10 15
<210> 40
   <211> 14
   <212> PRT
   <213> Hepatitis C virus
<400> 40
   Arg Ala Tyr Met Asn Thr Pro Gly Leu Pro Val Ala Gln Asp
   1 5 10
<210> 41
   <211> 15
   <212> PRT
   <213> Hepatitis C virus
<400> 41
   Ala Gln Gly Tyr Lys Val Leu Val Leu Asn Pro Ser Val Ala Ala
   1 5 10 15
<210> 42
   <211> 15
   <212> PRT
   <213> Hepatitis C virus
<400> 42
   Lys Val Leu Val Leu Asn Pro Ser Val Ala Ala Thr Leu Gly Phe
   1 5 10 15
<210> 43
   <211> 15
   <212> PRT
   <213> Hepatitis c virus
<400> 43
   Asn Pro Ser Val Ala Ala Thr Leu Gly Phe Gly Ala Tyr Met Ser
   1 5 10 15
<210> 44
   <211> 15
   <212> PRT
   <213> Hepatitis C virus
<400> 44
   Thr Leu Gly Phe Gly Ala Tyr Met Ser Lys Ala His Gly Ile Asp
   1 5 10 15
<210> 45
   <211> 15
   <212> PRT
   <213> Hepatitis C virus
<400> 45
   Pro Gln Asp Val Lys Phe Pro Gly Gly Gly Gln Ile Val Gly Gly
   1 5 10 15
<210> 46
   <211> 16
   <212> PRT
   <213> Hepatitis C virus
<400> 46
   Gly Gly Gly Gln Ile Val Gly Gly Val Tyr Leu Leu Pro Arg Arg Gly
   1 5 10 15
<210> 47
   <211> 15
   <212> PRT
   <213> Hepatitis C virus
<400> 47
   Gly Gly Val Tyr Leu Leu Pro Arg Arg Gly Pro Arg Leu Gly Val
   1 5 10 15
<210> 48
   <211> 15
   <212> PRT
   <213> Hepatitis C virus
<400> 48
   Arg Leu Gly Val Arg Ala Thr Arg Lys Thr Ser Glu Arg Ser Gln
   1 5 10 15
<210> 49
   <211> 15
   <212> PRT
   <213> Hepatitis C virus
<400> 49
   Thr Ala Gly Phe Ala Asp Leu Met Gly Tyr Ile Pro Leu Val Gly
   1 5 10 15
<210> 50
   <211> 15
   <212> PRT
   <213> Hepatitis C virus
<400> 50
   Ala Asp Leu Met Gly Tyr Ile Pro Leu Val Gly Ala Pro Leu Gly
   1 5 10 15
<210> 51
   <211> 15
   <212> PRT
   <213> Hepatitis C virus
<400> 51
   Met Gly Tyr Ile Pro Leu Val Gly Ala Pro Leu Gly Gly Ala Ala
   1 5 10 15
<210> 52
   <211> 14
   <212> PRT
   <213> Hepatitis C virus
<400> 52
   Gly Ala Pro Leu Gly Gly Ala Ala Arg Ala Leu Ala His Gly
   1 5 10
<210> 53
   <211> 15
   <212> PRT
   <213> Hepatitis C virus
<400> 53
   Ala Arg Ala Leu Ala His Gly Val Arg Val Leu Glu Asp Gly Val
   1 5 10 15
<210> 54
   <211> 15
   <212> PRT
   <213> Hepatitis C virus
<400> 54
   Ala His Gly Val Arg Val Leu Glu Asp Gly Val Asn Tyr Ala Thr
   1 5 10 15
<210> 55
   <211> 15
   <212> PRT
   <213> Hepatitis C virus
<400> 55
   Glu Asp Gly Val Asn Tyr Ala Thr Gly Asn Leu Pro Gly Ala Ser
   1 5 10 15
<210> 56
   <211> 15
   <212> PRT
   <213> Hepatitis C virus
<400> 56
   Ala Lys Ala Val Asp Phe Ile Pro Val Glu Asn Leu Glu Thr Thr
   1 5 10 15
<210> 57
   <211> 15
   <212> PRT
   <213> Hepatitis C virus
<400> 57
   Ala Val Asp Phe Ile Pro Val Glu Asn Leu Glu Thr Thr Met Arg
   1 5 10 15
<210> 58
   <211> 15
   <212> PRT
   <213> Hepatitis C virus
<400> 58
   Ile Pro Val Glu Asn Leu Glu Thr Thr Met Arg Ser Pro Val Phe
   1 5 10 15
<210> 59
   <211> 14
   <212> PRT
   <213> Hepatitis C virus
<400> 59
   Glu Asn Leu Glu Thr Thr Met Arg Ser Pro Val Phe Thr Asp
   1 5 10
<210> 60
   <211> 15
   <212> PRT
   <213> Hepatitis C virus
<400> 60
   Lys Asn Gln Val Glu Gly Glu Val Gln Ile Val Ser Thr Ala Ala
   1 5 10 15
<210> 61
   <211> 15
   <212> PRT
   <213> Hepatitis C virus
<400> 61
   Glu Gly Glu Val Gln Ile Val Ser Thr Ala Ala Gln Thr Phe Leu
   1 5 10 15
<210> 62
   <211> 15
   <212> PRT
   <213> Hepatitis C virus
<400> 62
   Gly Lys Ala Ile Pro Leu Glu Val Ile Lys Gly Gly Arg His Leu
   1 5 10 15
<210> 63
   <211> 15
   <212> PRT
   <213> Hepatitis C virus
<400> 63
   Pro Leu Glu Val Ile Lys Gly Gly Arg His Leu Ile Phe Cys His
   1 5 10 15
<210> 64
   <211> 15
   <212> PRT
   <213> Hepatitis C virus
<400> 64
   Glu Val Ile Lys Gly Gly Arg His Leu Ile Phe Cys His Ser Lys
   1               5                   10                  15
<210> 65
   <211> 15
   <212> PRT
   <213> Hepatitis C virus
<400> 65
   Arg His Leu Ile Phe Cys His Ser Lys Lys Lys Ala Asp Glu Leu
   1               5                   10                  15
<210> 66
   <211> 15
   <212> PRT
   <213> Hepatitis C virus
<400> 66
   Lys Pro Gly Ile Tyr Arg Phe Val Ala Pro Gly Glu Arg Pro Ser
   1               5                   10                  15
<210> 67
   <211> 14
   <212> PRT
   <213> Hepatitis C virus
<400> 67
   Arg Phe Val Ala Pro Gly Glu Arg Pro Ser Gly Met Phe Asp
   1               5                   10
<210> 68
   <211> 15
   <212> PRT
   <213> Hepatitis C virus
<400> 68
   His Leu Glu Phe Trp Glu Gly Val Phe Thr Gly Leu Thr His Ile
   1               5                   10                  15
<210> 69
   <211> 15
   <212> PRT
   <213> Hepatitis C virus
<400> 69
   Leu Thr His Ile Asp Ala His Phe Leu Ser Gln Thr Lys Gln Ser
   1   5 10 15
<210> 70
   <211> 15
   <212> PRT
   <213> Hepatitis C virus
<400> 70
   Glu Gly Val Phe Thr Gly Leu Thr His Ile Asp Ala His Phe Leu
   1 5 10 15
<210> 71
   <211> 15
   <212> PRT
   <213> Hepatitis C virus
<400> 71
   Thr Gly Leu Thr His Ile Asp Ala His Phe Leu Ser Gln Thr Lys
   1 5 10 15
<210> 72
   <211> 15
   <212> PRT
   <213> Hepatitis C virus
<400> 72
   Asp Ala His Phe Leu Ser Gln Thr Lys Gln Ser Gly Glu Asn Leu
   1 5 10 15
<210> 73
   <211> 15
   <212> PRT
   <213> Hepatitis C virus
<400> 73
   Arg Gly Leu Leu Gly Cys Ile Ile Thr Ser Leu Thr Gly Arg Asp
   1 5 10 15
<210> 74
   <211> 15
   <212> PRT
   <213> Hepatitis C virus
<400> 74
   Gly Cys Ile Ile Thr Ser Leu Thr Gly Arg Asp Lys Asn Gln Val
   1 5 10 15
<210> 75
   <211> 13
   <212> PRT
   <213> Influenza virus
<400> 75
   Pro Lys Tyr Val Lys Gln Asn Thr Leu Lys Leu Ala Thr
   1 5 10
<210> 76
   <211> 13
   <212> PRT
   <213> Homo sapiens
<400> 76
   Ala Ala Tyr Ala Ala Ala Lys Ala Ala Ala Leu Ala Ala
   1 5 10
<210> 77
   <211> 14
   <212> PRT
   <213> Homo sapiens
<400> 77
   Ala Lys Thr Ile Ala Tyr Asp Glu Glu Ala Arg Gly Leu Glu
   1 5 10
<210> 78
   <211> 16
   <212> PRT
   <213> Homo sapiens
<400> 78
   Thr Glu Arg Val Arg Leu Val Thr Arg His Ile Tyr Asn Arg Glu Glu
   1               5                   10                  15
<210> 79
   <211> 15
   <212> PRT
   <213> Homo sapiens
<400> 79
   Glu Ala Glu Gln Leu Arg Ala Tyr Leu Asp Gly Thr Gly Val Glu
   1               5                   10                  15
<210> 80
   <211> 20
   <212> PRT
   <213> Homo sapiens
<400> 80
   Glu Ser Trp Gly Ala Val Trp Arg Ile Asp Thr Pro Asp Lys Leu Thr
   1               5                   10                  15
   Gly Pro Phe Thr
               20
<210> 81
   <211> 14
   <212> PRT
   <213> Homo sapiens
<400> 81
   Ala Gly Asp Leu Leu Ala Ile Glu Thr Asp Lys Ala Thr Ile
   1               5                   10
<210> 82
   <211> 17
   <212> PRT
   <213> Homo sapiens
<400> 82
<210> 83
   <211> 15
   <212> PRT
   <213> Hepatitis C virus
<400> 83
   Asp Pro Asn Ile Arg Thr Gly Val Arg Thr Ile Thr Thr Gly Ser
   1 5 10 15
<210> 84
   <211> 15
   <212> PRT
   <213> Hepatitis c virus
<400> 84
   Arg Thr Gly Val Arg Thr Ile Thr Thr Gly Ser Pro Ile Thr Tyr
   1 5 10 15
<210> 85
   <211> 15
   <212> PRT
   <213> Hepatitis C virus
<400> 85
   Arg Thr Ile Thr Thr Gly Ser Pro Ile Thr Tyr Ser Thr Tyr Gly
   1 5 10 15
<210> 86
   <211> 15
   <212> PRT
   <213> Hepatitis C virus
<400> 86
   Pro Ile Thr Tyr Ser Thr Tyr Gly Lys Phe Leu Ala Asp Gly Gly
   1 5 10 15